(19) Europäisches Patentamt
European Patent Office
Office européen des brevets

(11) EP 3 131 849 B1

(12) EUROPEAN PATENT SPECIFICATION

(45) Date of publication and mention of the grant of the patent:
**15.08.2018 Bulletin 2018/33**

(21) Application number: **15714847.9**

(22) Date of filing: **14.04.2015**

(51) Int Cl.:
*C01B 32/05* (2017.01) *C01B 32/16* (2017.01)
*B01J 23/42* (2006.01) *B82Y 30/00* (2011.01)
*B82Y 40/00* (2011.01) *C07C 13/62* (2006.01)
*C07C 15/20* (2006.01) *C23C 16/26* (2006.01)
*C23C 16/44* (2006.01) *C01B 32/162* (2017.01)

(86) International application number:
**PCT/EP2015/058058**

(87) International publication number:
**WO 2015/158705 (22.10.2015 Gazette 2015/42)**

(54) **PROCESS FOR PREPARING SINGLE WALL CARBON NANOTUBES OF PRE-DEFINED CHIRALITY**

VERFAHREN ZUR HERSTELLUNG VON EINWANDIGEN KOHLENSTOFFNANORÖHRCHEN VON VORGEGEBENER CHIRALITÄT

PROCÉDÉ DE PRÉPARATION DE NANOTUBES DE CARBONE À PAROI SIMPLE AYANT UNE CHIRALITÉ PRÉDÉFINIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2014 EP 14164733**

(43) Date of publication of application:
**22.02.2017 Bulletin 2017/08**

(73) Proprietors:
- **Max-Planck-Gesellschaft zur Förderung der Wissenschaften e.V.**
  **80539 München (DE)**
- **EMPA Eidgenössische Materialprüfungs- und Forschungsanstalt**
  **8600 Dübendorf (CH)**

(72) Inventors:
- **FASEL, Roman**
  **CH-8057 Zürich (CH)**
- **RUFFIEUX, Pascal**
  **CH-1737 Plasselb (CH)**
- **SANCHEZ VALENCIA, Juan Ramon**
  **E-29016 Malaga (ES)**
- **JANSEN, Martin**
  **53127 Bonn (DE)**
- **MÜLLER, Andreas**
  **66287 Quierschied (DE)**
- **AMSHAROV, Konstantin**
  **91058 Erlangen (DE)**

(74) Representative: **Haggenmüller, Christian**
**Maiwald Patentanwalts- und Rechtsanwaltsgesellschaft mbH**
**Elisenhof**
**Elisenstraße 3**
**80335 München (DE)**

(56) References cited:
**EP-A1- 2 684 844** **WO-A1-2010/151307**

- **DAISUKE TAKAGI ET AL: "Single-Walled Carbon Nanotube Growth from Highly Activated Metal Nanoparticles", NANO LETTERS, vol. 6, no. 12, 1 December 2006 (2006-12-01), pages 2642-2645, XP055036207, ISSN: 1530-6984, DOI: 10.1021/nl061797g**
- **DEBOSRUTI DUTTA ET AL: "Epitaxial nucleation model for chiral-selective growth of single-walled carbon nanotubes on bimetallic catalyst surfaces", CARBON, ELSEVIER, OXFORD, GB, vol. 50, no. 10, 30 March 2012 (2012-03-30), pages 3766-3773, XP028509415, ISSN: 0008-6223, DOI: 10.1016/J.CARBON.2012.03.051 [retrieved on 2012-04-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- JASTI R ET AL: "Progress and challenges for the bottom-up synthesis of carbon nanotubes with discrete chirality", CHEMICAL PHYSICS LETTERS, ELSEVIER BV, NL, vol. 494, no. 1-3, 9 July 2010 (2010-07-09), pages 1-7, XP027108611, ISSN: 0009-2614, DOI: 10.1016/J.CPLETT.2010.04.067 [retrieved on 2010-05-17]

## Description

Single-Walled Carbon Nanotubes (SWCNTs) represent an extended family of quasi one-dimensional nanostructures with tubular shape, composed of $sp^2$-hybridised carbon atoms exclusively. Over the last decade SWCNTs have received tremendous attention from almost all areas of science because of their extraordinary thermal, mechanical, electronic and optical properties, and many potential high-technology applications. These materials are believed to be the best candidates that could revolutionize existing microelectronic by pushing it towards the nanometer scale. The diversity in properties which vary from semiconducting to conducting behaviour strongly depends on the orientation of the hexagonal lattice relative to the tube axis, as defined by the chiral indices (n, m) (chirality) and on the SWCNT diameter. Unfortunately, the wide application and exploring the potential of SWCNT for device performances are largely impeded by limited availability of uniform isomerically pure carbon nanotubes. SWCNTs are currently produced as highly heterogeneous mixture containing nanotubes of many different chiralities and diameters, regardless of the production technique used. Despite the considerable progress made in the past years in the development of sophisticated purification methods, a facile and effective separation of isomerically pure SWCNT suitable for large-scale technological processes remains still elusive.

One approach for preparing SWCNTs is by a vapour phase reaction of carbon-source molecules such as ethanol or ethylene on a catalyst surface (also referred to as chemical vapour deposition CVD). For initiating spontaneous reaction of the carbon-source molecules and, consequently, growth of SWCNTs on the catalyst surface, the catalyst particles need to have a diameter which corresponds more or less to the diameter of the SWCNTs to be prepared. Furthermore, these CVD processes are typically carried out at quite high temperature. Preparation of SWCNTs by vapour phase reaction needs the presence of nano-sized catalyst particles as described e.g. by Y. Homma et al., Nano Letters, Vol. 6, No. 12, 2006, pp. 2642-2645, and T. Maruyama et al., Mater. Express, Vol. 1, No. 4, 2011, pp. 267-272.

As discussed by R. Herges et al. in Chapter 10 ("Aromatic Belts as Sections of Nanotubes") of Fragments of Fullerenes and Carbon Nanotubes, 2012, John Wiley & Sons, several approaches for a template-based CNT synthesis have been developed such as starting from nanotube caps or nanotube belts. For CNT growing strategies, different approaches are mentioned including *inter alia* metal-catalyzed vapour phase reaction (CVD). It is described that the catalyst particles used in such metal-catalyzed vapour phase reactions have a diameter which is about the diameter of the carbon nanotube to be prepared.

US 2012/0177561 A1 describes the preparation of SWCNTs by converting a polycyclic aromatic compound to an end cap of a SWCNT, said end cap acting as a SWCNT precursor element which is then grown to the final SWCNT in a Diels-Alder reaction, which is carried out in a solvent, preferably in the absence of a metal catalyst.

K. Amsharov and A. Müller, Eur. J. Org. Chem., 2012, 6155-6164 describe the preparation of polycyclic aromatic compounds that might be useful for preparing SWCNT precursor elements. However, neither the preparation of the precursor elements nor the growth of precursor elements to SWCNTs is actually described.

EP 2 684 844 A1 describes a method for producing a carbon nanotube, comprising reacting a carbon source with a cyclic compound in which multiple aromatic rings are continuously bonded.

Daisuke Takagi et al., Nano Letters, Vol. 6, No. 12, December 2006, pp. 2642-2645, describe the preparation of single-wall carbon nanotubes using metal nanoparticles of 3 nm or less in diameter.

Debosruti Dutta et al., Carbon, Vol. 50, No. 10, March 2012, pp. 3766-3773, describe an epitaxial nucleation model for chiral-selective growth of single-wall carbon nanotubes on bimetallic catalyst surfaces.

It is an object of the present invention to provide single wall carbon nanotubes (SWCNTs) of pre-defined chirality in high isomeric purity.

According to a first aspect of the present invention, the object is solved by a process for preparing single wall carbon nanotubes (SWCNTs) having a diameter $d_{SWCNT}$, which comprises

(i) providing a precursor element which comprises a segment $S_{SWCNT}$ of the single wall carbon nanotube, wherein the precursor element is prepared from a polycyclic aromatic compound by a surface-catalyzed intramolecular cyclisation,
the segment $S_{SWCNT}$ being made of at least one ring formed by ortho-fused benzene rings, and having a first end E1 which is open and a second end E2 which is opposite to the first end E1,
the precursor element optionally further comprising a cap which is attached to the second end E2 of the segment $S_{SWCNT}$,
(ii) growing the precursor element by vapour phase reaction with a carbon-source compound on the surface of a metal-containing catalyst, wherein the precursor element is in contact with the surface of the metal-containing catalyst via the open end E1 of the segment $S_{SWCNT}$, and the metal-containing catalyst is in the form of particles having an average diameter $d_{cat}$ satisfying the following relation: $d_{cat} > 2 \times d_{SWCNT}$ or in the form of a continuous film, and wherein the metal-containing catalyst of step (ii) comprises a metal selected from Pd, Pt, Ru, Ir, Rh, Au, Ag, Fe, Co, Cu, Ni, or any mixture or alloy thereof.

In the present invention, it has surprisingly been realized that selective growth of a precursor element to SWCNTs of pre-defined chirality by vapor phase reaction does not need to be carried out in the presence of catalyst particles having a diameter comparable to the SWCNT diameter but can be efficiently accomplished in the presence of larger catalyst particles (which do not need to have a narrow particle size distribution) or even continuous catalyst films, if said precursor element comprises a nanotube segment $S_{SWCNT}$ being made of at least one ring formed by ortho-fused benzene rings, and the nanotube segment $S_{SWCNT}$ is brought into contact with the metal catalyst via its open end E1 while the other end of the nanotube segment $S_{SWCNT}$ can be open or closed by a SWCNT cap. As also discussed below, by using such a precursor element for vapor phase growth, it is not only possible to use CVD catalyst particles of larger size or even continuous catalyst films but also to improve yield at low growth temperature. Furthermore, using larger catalyst particles in step (ii) minimizes the risk or even excludes that the carbon-source compounds are spontaneously generating CNTs of undefined structure on the catalyst surface and makes sure that these carbon-source compounds are exclusively reacting with the SWCNT precursor element already provided in step (i).

As known to the skilled person, a nanotube is specified by the chiral vector

$\mathbf{C} = n\mathbf{a1}+m\mathbf{a2}$ which represents the rolling direction of the graphene sheet and hence the CNT chirality. Accordingly, the structure of a carbon nanotube can be represented by a pair of integers (n,m). If m=0, the carbon nanotubes are so-called zigzag carbon nanotubes. If n=m, the carbon nanotubes are so-called armchair carbon nanotubes. Otherwise, the carbon nanotubes are classified as chiral.

With the process of the present invention, it is possible to obtain any of these single wall carbon nanotube structures. In other words, the single wall carbon nanotubes (SWCNTs) of the present invention can be armchair single wall carbon nanotubes ((n,n)-SWCNTs), zig-zag single wall carbon nanotubes ((n,0)-SWCNTs) or chiral single wall carbon nanotubes ((n,m)-SWCNTs with $n \neq m$).

If the SWCNTs are armchair single wall carbon nanotubes (i.e. (n,n)-SWCNTs), the integer n can vary over a broad range, e.g. from 2 to 30, preferably from 3 to 20, or from 5 to 15.

If the SWCNTs are zig-zag single wall carbon nanotubes (i.e. (n,0)-SWCNTs), the integer n can vary over a broad range, e.g. from 3 to 50, preferably from 4 to 35, or from 5 to 15.

If the SWCNTs are chiral single wall carbon nanotubes (i.e. (n,m)-SWCNTs, wherein $n \neq m$), each of the integers n and m can vary over a broad range, e.g. $5 \leq n+m \leq 60$; or $6 \leq n+m \leq 20$.

As indicated above, in step (i) of the process of the present invention, a precursor element of the single wall carbon nanotube is provided, wherein the precursor element comprises a segment $S_{SWCNT}$ of the single wall carbon nanotube, the segment $S_{SWCNT}$ being made of at least one ring formed by ortho-fused benzene rings and having a first end E1 which is open and a second end E2 which is opposite to the first end E1.

This segment $S_{SWCNT}$ can also be referred to as a nanotube belt. If many of these segments $S_{SWCNT}$ were attached to each other via their ends, they would form the predefined single wall carbon nanotube.

The second end E2 of the segment $S_{SWCNT}$ can be open as well. Alternatively, the precursor element further comprises a cap of the single wall carbon nanotube, the cap being attached to the second end E2 of the segment $S_{SWCNT}$ (i.e. thereby resulting in a nanotube segment $S_{SWCNT}$ having an open end E1 and a closed end E2).

The one or more rings the carbon nanotube segment $S_{SWCNT}$ is made of are formed by ortho-fused benzene rings. A system in which any two adjacent aromatic rings have two, and only two, adjacent atoms in common are said to be '*ortho*-fused'. If the first benzene ring and the final benzene ring in a sequence of ortho-fused benzene rings are also fused to each other, a segment ring of the carbon nanotube segment $S_{SWCNT}$ is formed. The fused benzene rings forming the segment ring can all be in a linear sequence (such a ring also referred to as a cyclacene) or an angular sequence (such a ring also referred to as a cyclophenacene), or some of the fused benzene rings forming the segment ring are in a linear sequence whereas other fused benzene rings in the same segment ring are in an angular sequence. The segment $S_{SWCNT}$ may have further segment rings (each segment ring being formed by ortho-fused benzene rings). If two or more segment rings are present and neighbouring segment rings are fused to each, this means that the "upper rim" of the first ring and the "lower rim" of the neighbouring ring are formed by the same carbon atoms. However, it is also possible that the segment $S_{SWCNT}$ is just made of a single segment ring. Furthermore, it is also possible that the segment $S_{SWCNT}$ is not terminated, either at one of its ends E1 or E2 or at both ends, by a complete segment ring, of course under the provision that at least one segment ring formed by ortho-fused benzene rings is present.

Preferably, the segment $S_{SWCNT}$ is made of (i.e. consists of) from 1 to 10 segment rings, each segment ring being formed by ortho-fused benzene rings.

The precursor element is prepared from a polycyclic aromatic compound.

As known to the skilled person, a polycyclic aromatic compound is a compound containing fused aromatic rings as a structural element.

In a preferred embodiment, the precursor element is made of the segment $S_{SWCNT}$ and a cap of the SWCNT to be prepared, the cap being attached to the second end E2 of the segment $S_{SWCNT}$ (thereby resulting in a segment $S_{SWCNT}$ having a closed end E2 and an open end E1).

It is known to the skilled person that end caps (or parts thereof) of SWCNTs can be prepared from polycyclic

aromatic compounds. Reference can be made to US 2012/0177561 A1 and K.Y. Amsharov, A. Müller, Eur. J. Org. Chem., 2012, pp. 6155-6164. In the present invention, polycyclic aromatic compounds are preferably used which, if subjected to an intramolecular cyclization, do not just provide the end cap itself of the pre-defined SWCNT but an "extended end cap" additionally containing a carbon nanotube segment $S_{SWCNT}$ (i.e. at least one ring formed by ortho-fused benzene rings) attached to the cap.

If the segment $S_{SWCNT}$ has an end E2 which is closed by a SWCNT cap, the number of segment rings can be e.g. from 1 to 10. However, as complexity of the organic synthesis of polycyclic aromatic compounds may increase with increasing molecular size, the number of segment rings is preferably kept low, e.g. 1 to 3 segment rings or 1 to 2 segment rings or even just one segment ring.

Figure 1 shows the structure of an exemplary precursor element from which a (6,6) single wall carbon nanotube can be prepared. In this exemplary structure, the precursor element is not only made of a SWCNT segment (gray shading) but additionally contains a cap at one end of the SWCNT segment. The SWCNT segment (gray-shaded area) is made of segment rings, each of these segment rings being formed by ortho-fused benzene rings.

The polycyclic aromatic compound used for preparing the precursor element (which is preferably made of the segment $S_{SWCNT}$ and a SWCNT cap attached to the second end E2 of the segment $S_{SWCNT}$) can be e.g. a compound of formula (1) having a central benzene ring with residues $R^1$ to $R^6$

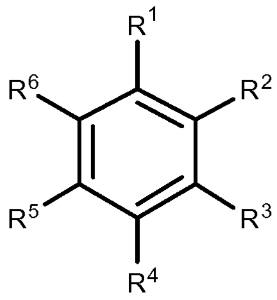

(1)

wherein

each of the residues $R^1$, $R^3$ and $R^5$, which can be the same or different, has the following formula (2)

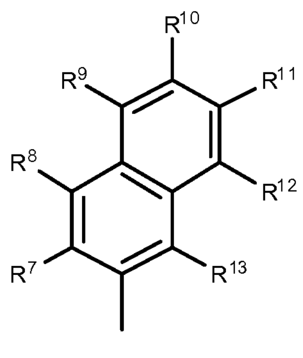

(2)

wherein

at least one of the residues $R^7$ to $R^{13}$ is a substituted or unsubstituted phenyl or a polycyclic aromatic group; or
at least two of the residues $R^7$ to $R^{13}$ which are adjacent to each other form together a monocyclic or polycyclic aromatic group;

the residues $R^2$, $R^4$ and $R^6$, which can be the same or different, are hydrogen; or have the following formula (3)

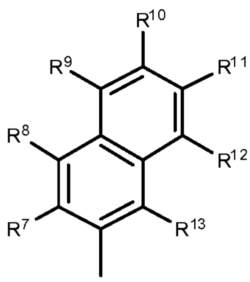

(3)

wherein $R^7$ to $R^{13}$ have the same meaning as in formula (2); or represent a chemical bond connecting the central benzene ring with the residue $R^7$ or $R^{13}$ in formula (2).

As already mentioned above, the residues $R^1$, $R^3$ and $R^5$ can be identical or different from each other. The same is true for the residues $R^2$, $R^4$ and $R^6$. Furthermore, if present, the residues of formula (3) can be identical with or different from the residues of formula (2).

Optionally, if one or more of the residues $R^2$, $R^4$ and $R^6$ are of formula (3), it is possible that $R^7$ in one of the residues $R^1$, $R^3$, and $R^5$ of formula (2) and $R^{13}$ in one of the adjacent residues $R^2$, $R^4$, and $R^6$ of formula (3), and/or $R^{13}$ in one of the residues $R^1$, $R^3$, and $R^5$ of formula (2) and $R^7$ in one of the adjacent residues $R^2$, $R^4$, and $R^6$ of formula (3) represent/form together a chemical bond.

If one of the residues $R^7$ to $R^{13}$ in formula (2) is a substituted phenyl, the one or more substituents can be e.g. a phenyl group. The same is true for the residues $R^7$ to $R^{13}$ in formula (3.)

If one of the residues $R^7$ to $R^{13}$ in formula (2) is a polycyclic aromatic group, the number of fused benzene rings in said polycyclic aromatic group can vary e.g. from 2 to 8, preferably from 2 to 6 or from 2 to 4. The same is true for the residues $R^7$ to $R^{13}$ in formula (3.)

If at least two of the residues $R^7$ to $R^{13}$ of formula (2) which are adjacent to each other form together a monocyclic aromatic group, it is preferably a six-membered aromatic ring. The same is true for the residues $R^7$ to $R^{13}$ in formula (3.)

If at least two of the residues $R^7$ to $R^{13}$ of formula (2) which are adjacent to each other form together a polycyclic aromatic group, the number of fused benzene rings in said polycyclic aromatic group can vary e.g. from 2 to 8, preferably from 2 to 6 or from 2 to 4. The same is true for the residues $R^7$ to $R^{13}$ in formula (3.)

If the polycyclic aromatic group is formed by two fused benzene rings, it is derived from naphthalene, which can be substituted or unsubstituted.

If the polycyclic aromatic group is formed by three fused benzene rings, it is derived from phenanthrene or anthracene, each of which can be substituted or unsubstituted.

If the polycyclic aromatic group is formed by four fused benzene rings, it is derived from e.g. chrysene, tetraphene (i.e. benz[a]anthracene), tetracene, triphenylene (i.e. 9,10-benzophenanthrene), benzo[c]phenantrene, or pyrene, each of which can be substituted or unsubstituted.

If the polycyclic aromatic group is formed by five fused benzene rings, it is derived from e.g. dibenzo-anthracenes such as dibenzo[a,h]anthracene, dibenzo[a,c]anthracene, 1,2:7,8-dibenzanthracene (also known as dibenz[a,j]anthracene and 1,2:5,6-dibenzoanthracene; benzo-chrysenes such as picene (also known as benzo[a]chrysene) and benzo[b]chrysene; pentacene; dibenzo-phenanthrenes such as 1,2:3,4-dibenzophenanthrene and 3,4,5,6-dibenzophenantrene; benzo-naphthacenes such as benzo[a]naphthacene; benzo-pyrenes such as benzo[a]pyrene and benzo[e]pyrene; each of which can be substituted or unsubstituted.

If the polycyclic aromatic group is formed by six fused benzene rings, it is derived from e.g. benzo-picenes such as benzo[s]picene; benzo-pentacenes; benzo-perylenes; or dibenzo-pyrenes; each of which can be substituted or unsubstituted.

Alternatively, the polycyclic aromatic compound used for preparing the precursor element (which is preferably made of the segment $S_{SWCNT}$ and a SWCNT cap attached to the second end E2 of the segment $S_{SWCNT}$) can be e.g. a compound of formula (4)

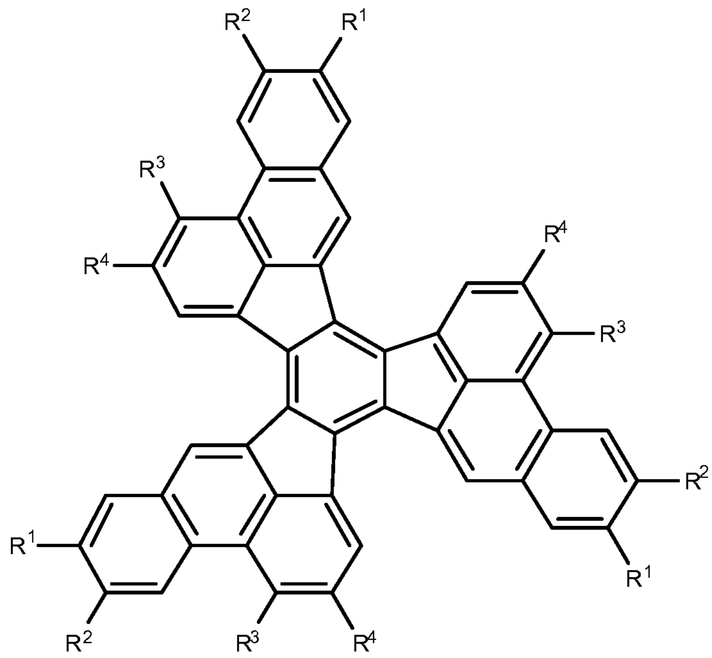

(4)

wherein

at least one of the residues $R^1$ to $R^4$ is a substituted or unsubstituted phenyl or a polycyclic aromatic group; or the residues $R^1$ and $R^2$ and/or the residues $R^3$ and $R^4$ form together a monocyclic or polycyclic aromatic group.

With regard to exemplary polycyclic aromatic groups, reference can be made to the statements provided above.

If one or more of the residues $R^1$ to $R^4$ is/are a substituted phenyl, the one or more substituents can be e.g. phenyl.

In formula (4), the residues $R^1$ and $R^2$, independently from each other, can be e.g. hydrogen, a substituted or unsubstituted phenyl or a polycyclic aromatic group; and the residues $R^3$ and $R^4$ may form together a benzene ring carrying a phenyl or biphenyl substituent, or the residues $R^3$ and $R^4$ may form together a polycyclic aromatic group of 2 to 6 fused benzene rings, which can be substituted or unsubstituted.

Alternatively, the polycyclic aromatic compound used for preparing the precursor element (which is preferably made of the segment $S_{SWCNT}$ and a SWCNT cap attached to the second end E2 of the segment $S_{SWCNT}$) can be e.g. a corannulene compound.

An exemplary polycyclic aromatic compound that can be used for preparing (6,6) armchair SWCNTs has one of the following formulas (I) and (II):

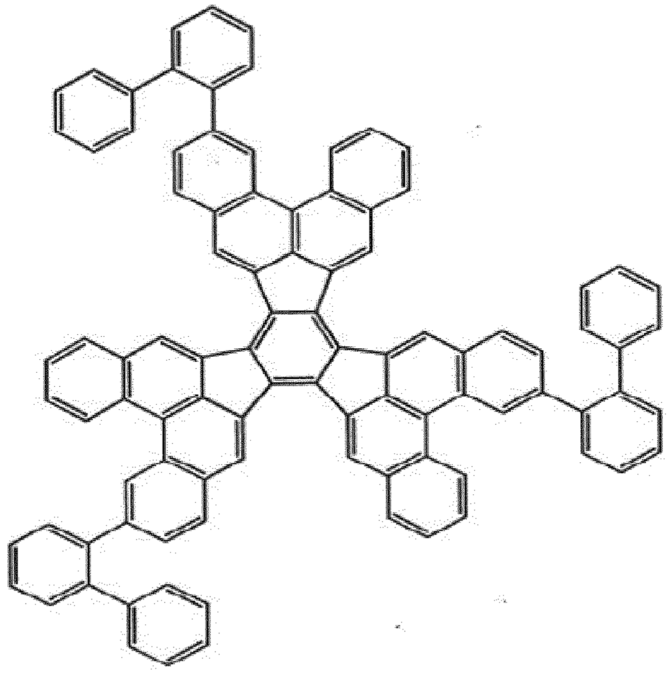

Formula (I)

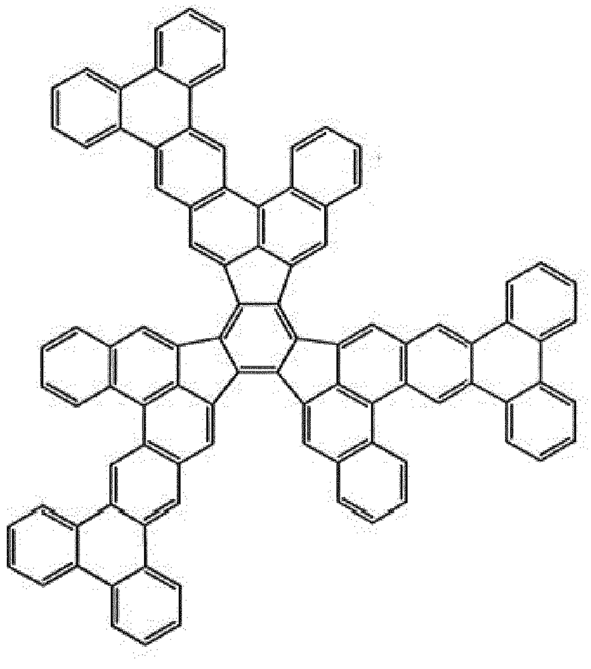

Formula (II)

An exemplary polycyclic aromatic compound that can be used for preparing (9,9) armchair SWCNTs has the following formula (III):

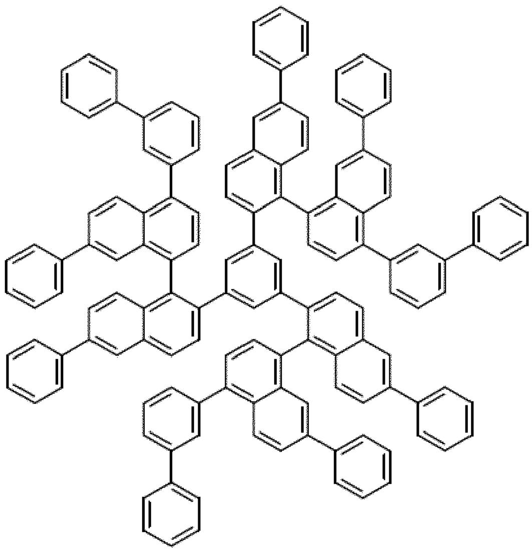

Formula (III)

An exemplary compound that can be used for preparing (9,0) zig-zag SWCNTs has the following formula (IV):

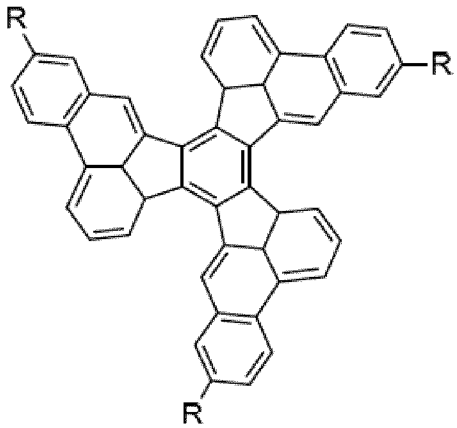

Formula (IV)

wherein R is phenyl (i.e. $-C_6H_5$).

An exemplary compound that can be used for preparing (12,6) chiral SWCNTs has one of the following formulas (V) and (VI):

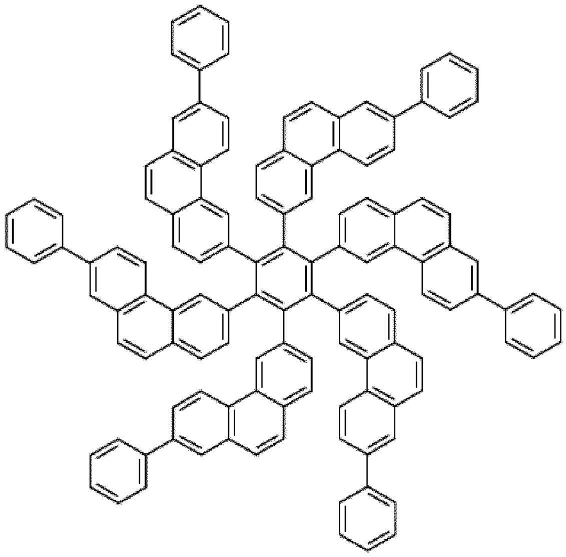

Formula (V)

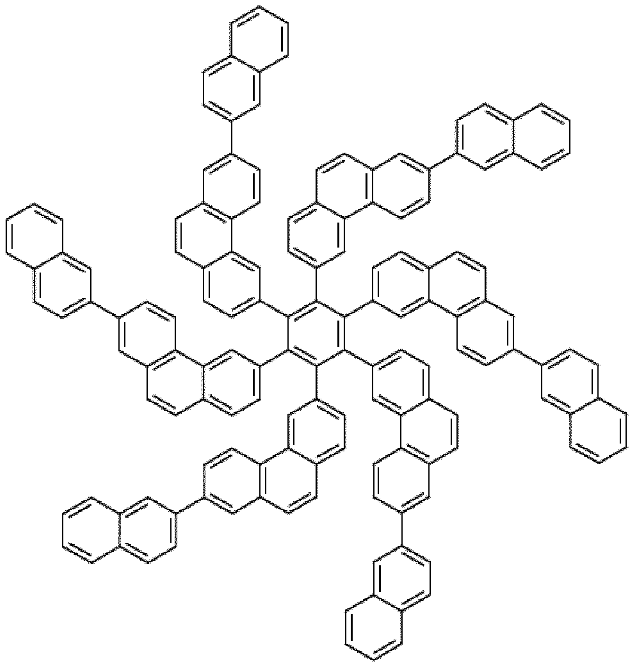

Formula (VI)

An exemplary compound that can be used for preparing (12,0) zig-zag SWCNTs has one of the following formulas (VII) and (VIII):

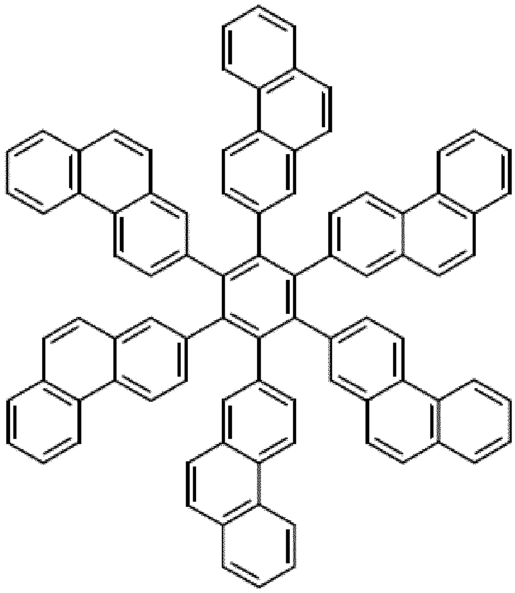

Formula (VII)

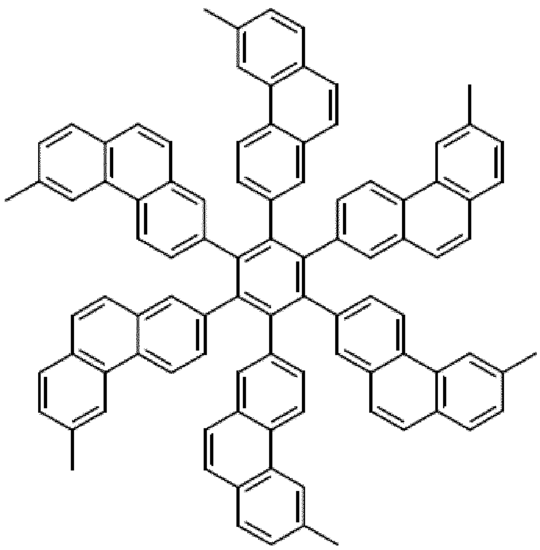

Formula (VIII)

An exemplary compound that can be used for preparing (12,3) chiral SWCNTs has the following formula (IX):

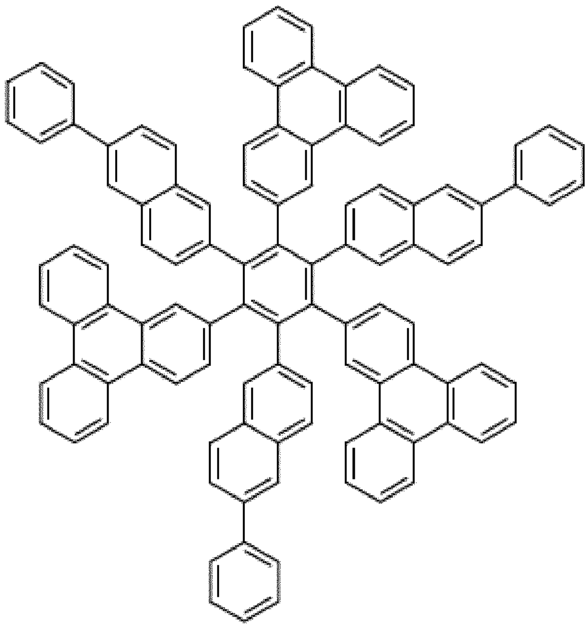

Formula (IX)

An exemplary compound that can be used for preparing (12,1) chiral SWCNTs has the following formula (X):

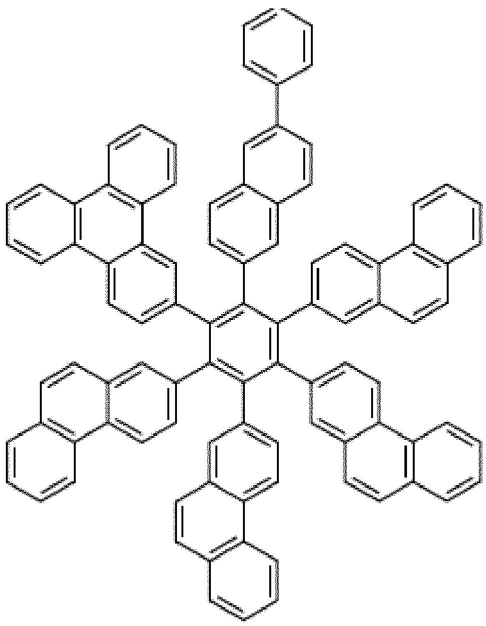

Formula (X)

An exemplary compound that can be used for preparing (11,3) chiral SWCNTs has the following formula (XI):

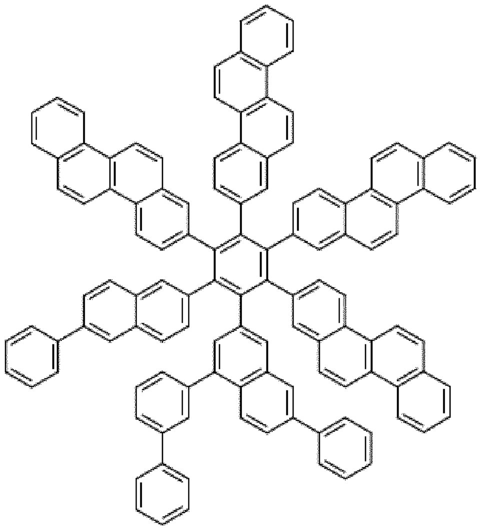

Formula (XI)

An exemplary compound that can be used for preparing (12,2) chiral SWCNTs has the following formula (XII):

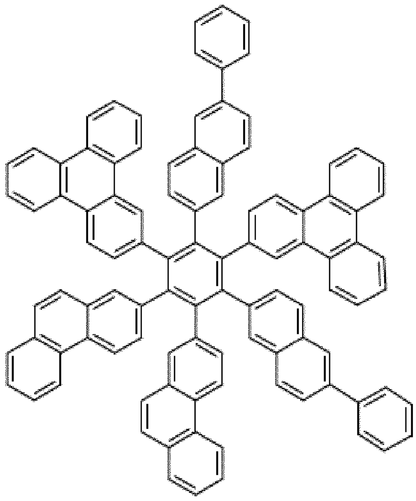

Formula (XII)

An exemplary compound that can be used for preparing (10,6) chiral SWCNTs has the following formula (XIII):

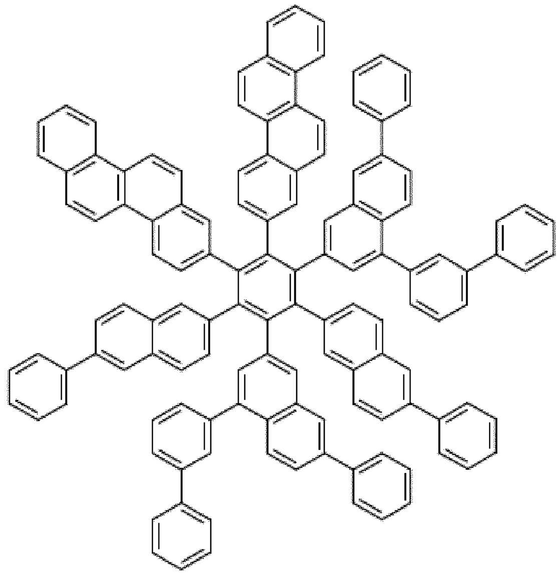

Formula (XIII)

An exemplary compound that can be used for preparing (11,5) chiral SWCNTs has the following formula (XIV):

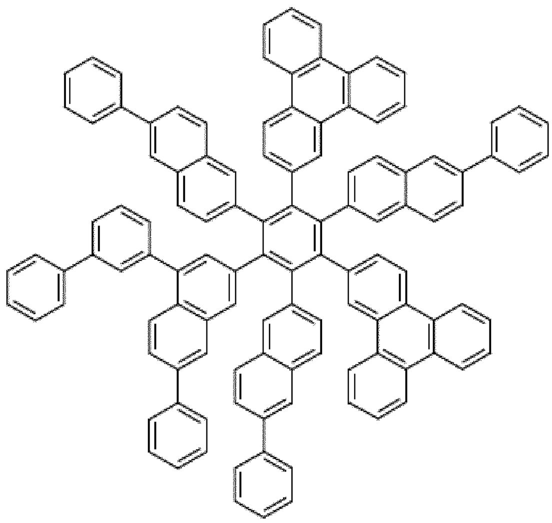

Formula (XIV)

An exemplary compound that can be used for preparing (10,7) chiral SWCNTs has the following formula (XV):

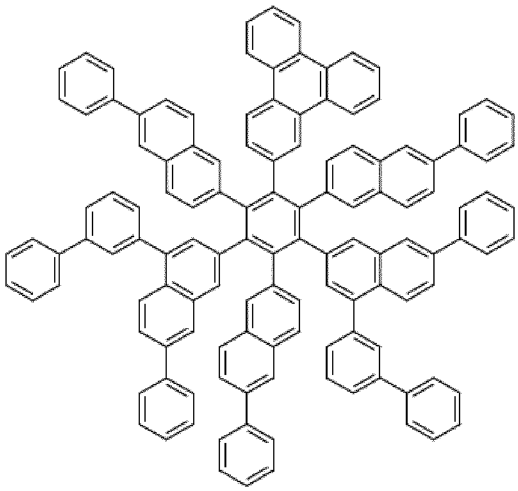

Formula (XV)

An exemplary compound that can be used for preparing (11,4) chiral SWCNTs has the following formula (XVI):

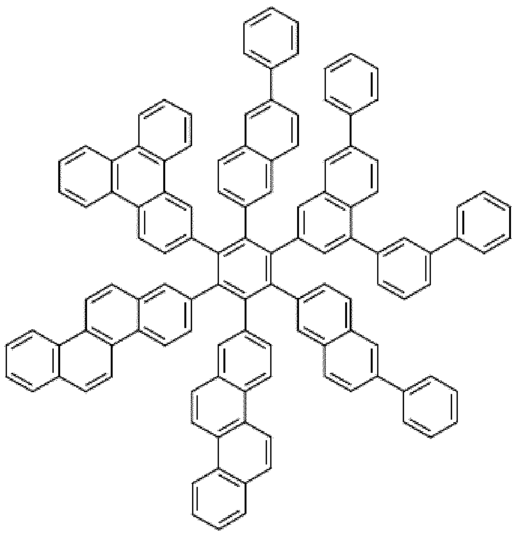

Formula (XVI)

An exemplary compound that can be used for preparing (8,2) chiral SWCNTs has the following formula (XVII):

(8, 2)

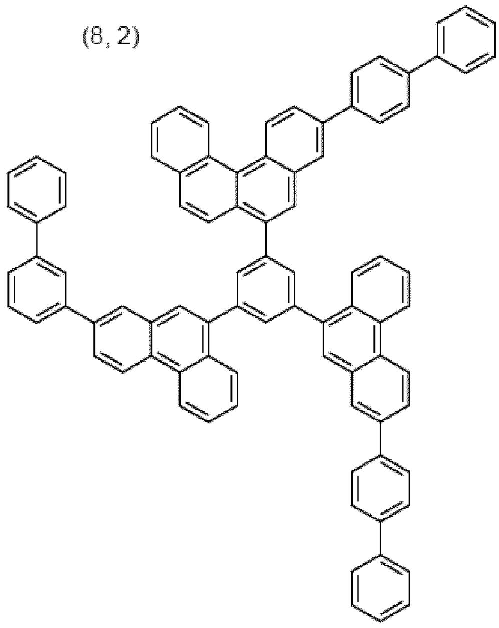

Formula (XVII)

An exemplary compound that can be used for preparing (7,4) chiral SWCNTs has the following formula (XIII):

(7, 4)

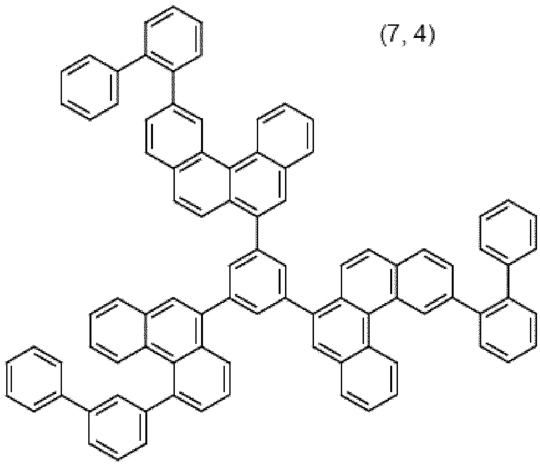

Formula (XVIII)

An exemplary compound that can be used for preparing (11,0) zig-zag SWCNTs has the following formula (XIX):

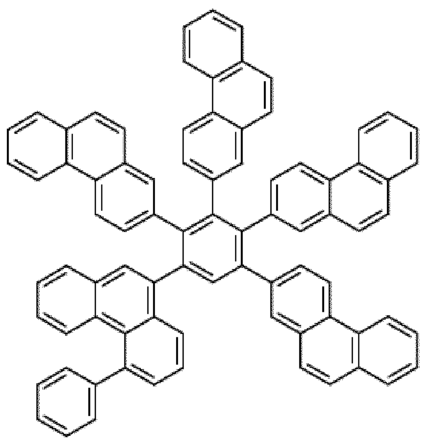

Formula (XIX)

An exemplary compound that can be used for preparing (10,2) chiral SWCNTs has the following formula (XX):

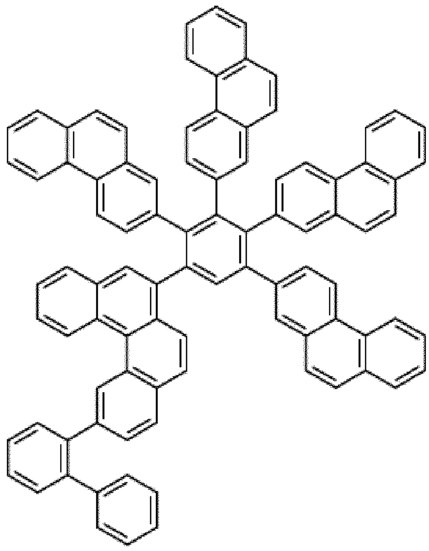

Formula (XX)

An exemplary compound that can be used for preparing (7,7) armchair SWCNTs has the following formula (XXI):

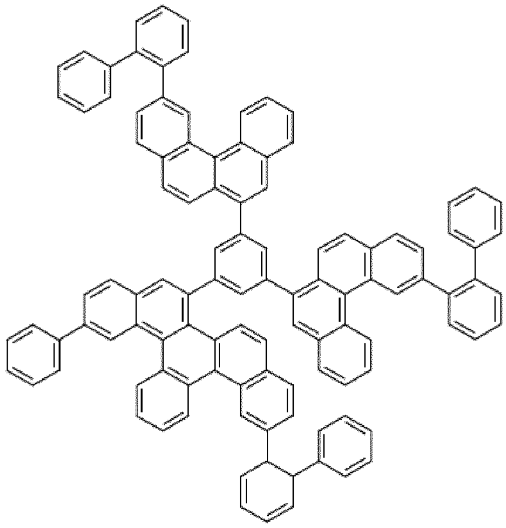

Formula (XXI)

An exemplary compound that can be used for preparing (8,8) armchair SWCNTs has the following formula (XXII):

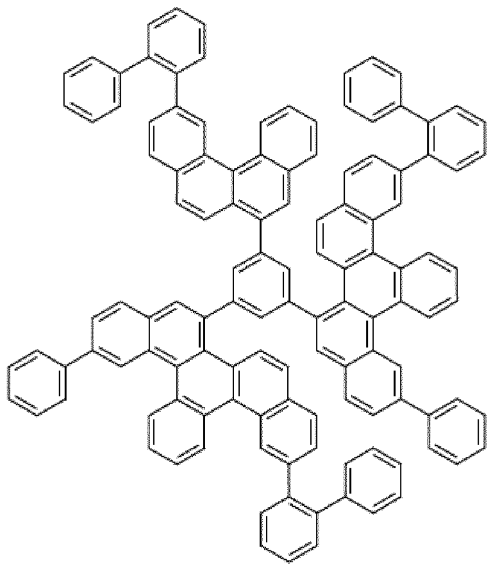

Formula (XXII)

An exemplary compound that can be used for preparing (9,5) armchair SWCNTs has the following formula (XXIII):

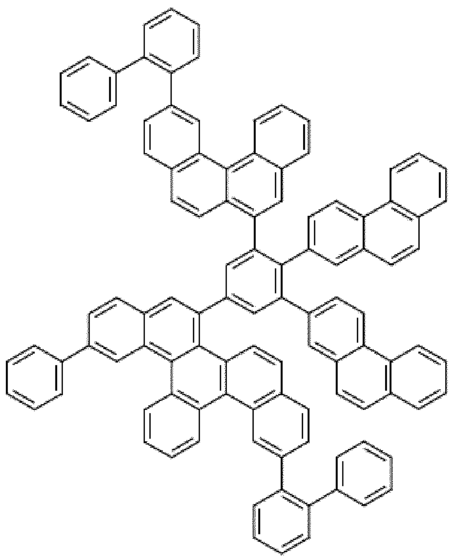

Formula (XXIII)

In any of these compounds (I) to (XXIII), at least some of the hydrogen atoms can be substituted by halide atoms (such as Cl, Br, or I).

If subjected to an intramolecular cyclization, each of the polycyclic aromatic compounds (I) to (XXIII) forms a precursor element which consists of a SWCNT segment $S_{SWCNT}$ and a SWCNT cap attached to one end of the segment. As discussed above, the segment $S_{SWCNT}$ of each of the precursor elements is made of at least one ring formed by ortho-fused benzene rings and has an open end E1.

The polycyclic aromatic compound can be prepared by organic synthesis procedures principally known to the skilled person.

Just as an example, the synthetic routes to the polycyclic aromatic compounds (I) and (II) are illustrated in Figures 2 and 3.

The synthetic route shown in Figure 2 for preparing the polycyclic aromatic compound of formula (I) (in Figure 2: compound 7) includes the following steps:

a) $PPh_3$, toluene, reflux, 95%; b) $BrPh_3PCH_2PhBr$, KOtBu, EtOH, reflux, 81%; c) $I_2$, *hv,* propylene oxide, cyclohexane, 72%; d) $Pd(PPh_3)_4$, $Cs_2CO_3$, toluene/MeOH, 110°C, 79%; e) NBS, DBPO, $CCl_4$, reflux, 70%; f) NaCN, DMSO, RT,

40%; g) $H_2SO_4$, $H_2O$, HOAc, reflux, 98%; h) $SOCl_2$, 65 °C; i) $AlCl_3$, $CH_2Cl_2$, RT, 57%; j) propanoic acid, TsOH, o-DCB, 180°C, 65%.

The synthetic route shown in Figure 3 for preparing the polycyclic aromatic compound of formula (II) (in Figure 3: compound 19) includes the following steps:

a) $Pd(PPh_3)_4$, $K_2CO_3$, toluene/MeOH, 110°C, 86%; b) $I_2$, *hv,* propylene oxide, cyclohexane, 52%; c) NBS, DBPO, $CCl_4$, reflux, 87%; d) $PPh_3$, toluene, reflux, 71%; e) $P(OEt)_3$, 160°C, 60%; f) $C_{10}H_7COCH_3$, KOtBu, THF, 50°C, 31%; g) $I_2$, *hv,* propylene oxide, cyclohexane, 24%; h) NBS, DBPO, $CCl_4$, reflux, 60%; i) NaCN, DMSO, RT, 75%; j) $H_2SO_4$, $H_2O$, HOAc, reflux, 73%; k) $SOCl_2$, 65 °C; 1) $AlCl_3$, $CH_2Cl_2$, RT, 36%; m) $TiCl_4$, *o*-DCB, 180°C, 35%.

The precursor element is prepared from the polycyclic aromatic compound by intramolecular cyclization. Preferred intramolecular cyclization reactions are cyclodehydrogenation, cyclodehalogenation and Bergman cyclization.

When the polycyclic aromatic compound is subjected to a conversion step such as cyclodehydrogenation, aromatic rings of the polycyclic aromatic compound get connected via an intramolecular reaction and the aromatic compound, which can typically be considered as a more or less flat molecule, is converted to a "bowl-like" SWCNT precursor element made of the tube segment $S_{SWCNT}$ and the SWCNT cap closing one end of the tube segment while the other end of the tube segment remains open.

The intramolecular cyclization such as cyclodehydrogenation which is used for converting the polycyclic aromatic compound to the precursor element is a surface-assisted (also referred to as "surface-catalyzed") intramolecular cyclization. Appropriate process conditions and surfaces on which the polycyclic aromatic compound can be subjected to an intramolecular cyclization such as cyclodehydrogenation are known to the skilled person. In this context, reference can be made e.g. to K. Amsharov et al., Angew. Chem. Int. Ed. 2010, pp. 9392-9396 and G. Otero et al., Nature, Vol. 454, 2008, pp. 865-868.

In a preferred embodiment, the surface-assisted intramolecular cyclization is carried out on the surface of a metal-containing catalyst, more specifically on a metal surface of the metal-containing catalyst. Preferably, the metal is Pd, Pt, Ru, Ir, Rh, Au, Ag, Fe, Co, Cu, Ni, or any mixture or alloy thereof.

The polycyclic aromatic compound can be deposited on the catalyst surface by any process suitable for deposition of organic compounds on a surface. The process may e.g. be a vacuum deposition (sublimation) process, a solution based process such as spin coating, spray coating, dip coating, printing, electrospray deposition, pulsed jet deposition, dry contact transfer or dry imprint, a laser induced desorption process, or preparative mass spectrometry (especially for deposition of compounds which are only available in impure mixtures).

If the polycyclic aromatic compound comprises groups that may rotate about a single bond (e.g. a phenyl or biphenyl residue at the outer periphery of the molecule) and therefore shows conformational isomerism, the molecules deposited on the catalyst surface may have different conformations. However, exclusively those molecules that are adsorbed on the catalyst surface in an appropriate conformation will form (e.g. by cyclodehydrogenation) the SWCNT precursor element, which in turn will then grow to the final SWCNT in the vapour phase reaction of step (ii). Any molecule adsorbed in an inappropriate conformation will not form a SWCNT precursor element that may subsequently grow in step (ii). So, only those SWCNTs having the pre-defined chirality will actually be formed in step (ii). In other words, with the process of the present invention, SWCNTs of pre-defined chirality can be obtained in extremely high purity (i.e. purity of about 100%).

Preferably, the polycyclic aromatic compounds are deposited under conditions so as to achieve a surface density (i.e. number of compounds deposited per unit area of catalyst surface) which is high enough for optimizing yield of precursor elements and, consequently, SWCNTs, but low enough for suppressing contact between neighbouring compounds as much as possible. If two or more neighbouring polycyclic aromatic compounds come into contact, the yield of defined precursor elements obtained after the conversion step (i) may decrease.

For maximizing the contact area between the polycyclic aromatic compound and the catalyst surface and thereby improving efficiency of the cyclodehydrogenation step, the average diameter of the metal-containing catalyst particles should be kept sufficiently large.

Preferably, the metal-containing catalyst of step (i) is in the form of particles having an average diameter $d_{cat}$ satisfying the following relation: $d_{cat} > 2 \times d_{SWCNT}$, more preferably: $d_{cat} > 4 \times d_{SWCNT}$; even more preferably: $d_{cat} > 10 \times d_{SWCNT}$ or $d_{cat} > 20 \times d_{SWCNT}$ or $d_{cat} > 50 \times d_{SWCNT}$, or in the form of a continuous film.

The particles of the metal-containing catalyst in step (i) may have an average particle size of e.g. at least 5 nm, more preferably at least 10 nm, even more preferably at least 20 nm or at least 50 nm or at least 100 nm, under the provision that $d_{cat} > 2 \times d_{SWCNT}$.

The average particle size can be determined by an image analysis (e.g. images taken from transmission electron microscopy (TEM) or scanning electron microscopy (SEM)). In this image-based particle size analysis, the size distribution of the particles shown on the TEM (or SEM) image is determined. The particle size of an individual particle is its largest

extension as available from the image. The peak value of the size distribution curve is the average particle size.

The term "catalyst in the form of a continuous film" means that the dimensions of the surface provided by the catalyst and on which the polycyclic aromatic compounds are deposited, are by far exceeding the molecular size of the aromatic compound. Preferably, such a catalyst in the form of a film provides a flat surface, thereby improving interaction between the polycyclic aromatic compound and the catalyst surface and increasing efficiency of the intramolecular cyclization.

The catalyst used in step (i) can be crystalline, either partly or completely. However, it is also possible that the catalyst in step (i) is amorphous.

Catalysts that can be used in step (i) for intramolecular cyclization, are generally known to the skilled person and are commercially available or can be prepared by commonly known standard methods.

As known to the skilled person, the diameter of a SWCNT can be calculated from the following equation:

$$d_{SWCNT} = (n^2 + m^2 + nm)^{1/2} \text{ x } 0.0783 \text{ nm}$$

wherein n and m are the integers defining the chiral vector and therefore the structure of the SWCNT. Furthermore, as already discussed above, by selecting an appropriate polycyclic aromatic compound, a specific SWCNT with characteristic n,m-integers is obtained. From these known integers n and m, the diameter of the SWCNT can be calculated.

Appropriate process conditions for converting the polycyclic aromatic compound to the SWCNT precursor element in step (i) are principally known to the skilled person.

The temperature at which the intramolecular cyclization is carried out may vary over a broad range. Preferably, step (i) is carried out at a temperature $T_1$ of from 100°C to 1000°C, more preferably of from 200 to 800°C or from 200 to 600°C.

Alternatively, instead of using a nanotube segment $S_{SWCNT}$ which has an open end E1 and a closed end E2, it is also possible to use a nanotube segment $S_{SWCNT}$ which is open at both ends.

Such short nanotube segments which are open at both ends can be prepared chemically, e.g. via chemical reactions described in Pure Appl. Chem., 2012, Vol. 84, No. 4, pp. 907-916. Alternatively, a SWCNT be cut into smaller segments, e.g. electron beam lithography and oxygen plasma ion etching (see e.g. Nano Letters, 2009, Vol. 9, No. 4, pp. 1673-1677).

As indicated above, the process of the present invention comprises a step (ii) which includes growing the precursor element by vapour phase reaction with a carbon-source compound on the surface, preferably the metal surface, of a metal-containing catalyst, wherein the precursor element is in contact with the surface of the metal-containing catalyst via the open end E1 of the segment $S_{SWCNT}$, and the metal-containing catalyst is in the form of particles having an average diameter $d_{cat}$ satisfying the following relation: $d_{cat} > 2 \text{ x } d_{SWCNT}$ or in the form of a continuous film.

The carbon-source compound (i.e. a compound containing one or more carbon atoms) can be selected from those compounds which are typically used for manufacturing carbon nanotubes by chemical vapour deposition (CVD). Such carbon-source compounds are generally known to the skilled person.

A carbon-source compound that can be used in step (ii) of the present invention is e.g. an alkane such as a $C_{1-4}$ alkane (preferably methane or ethane), an alkene such as ethylene, an alkyne such as acetylene, an alcohol such as a $C_{1-4}$ alcohol (preferably methanol or ethanol), an aromatic compound such as benzene, carbon monoxide, nitrogen-containing organic compounds (e.g. amines), boron-containing organic compounds, or any mixture thereof. The carbon-source compound can be mixed with heteroatom-containing non-carbon compounds (e.g. $NH_3$, ... etc.) which may dope the SWCNT with heteroatoms.

In addition to the carbon-source compound, one may use one or more support gases such as $H_2$ or $H_2O$. The use of such support gases in a manufacturing process of carbon nanotubes via vapour phase reaction is generally known to the skilled person.

The metal of the metal-containing catalyst is Pd, Pt, Ru, Ir, Rh, Au, Ag, Fe, Co, Cu, Ni, or any mixture or alloy thereof.

The SWCNT precursor element prepared in step (i) can be brought into contact and deposited on the surface of the metal-containing catalyst by methods commonly known to the skilled person. If a metal-containing catalyst is used in step (i) and is different from the metal-containing catalyst of step (ii), the precursor elements can be transferred to the surface of the metal-containing catalyst of step (ii) by commonly known methods. However, as will be discussed below in further detail, it is preferred to use the same metal-containing catalyst in steps (i) and (ii). In this preferred embodiment, no transfer of the SWCNT precursor elements from the first catalyst to the second catalyst is needed. In other words, in this preferred embodiment, step (ii) includes growing the precursor element by vapour phase reaction with the carbon-source compound on the surface of the metal-containing catalyst of step (i).

Preferably, the metal-containing catalyst of step (ii) is in the form of particles having an average diameter $d_{cat}$ satisfying the following relation: $d_{cat} > 4 \text{ x } d_{SWCNT}$, more preferably: $d_{cat} > 10 \text{ x } d_{SWCNT}$ or $d_{cat} > 20 \text{ x } d_{SWCNT}$ or $d_{cat} >$

50 x $d_{SWCNT}$.

As mentioned above, it is known to the skilled person that the diameter of a SWCNT can be calculated from the following equation:

$$d_{SWCNT} = (n^2 + m^2 + nm)^{1/2} \times 0.0783 \text{ nm}$$

wherein n and m are the integers defining the chiral vector and therefore the structure of the SWCNT. Furthermore, by selecting an appropriate polycyclic aromatic compound, a specific SWCNT with characteristic n,m-integers is obtained. From these known integers n and m, the diameter of the SWCNT can be calculated.

The particles of the metal-containing catalyst in step (ii) may have an average particle size of e.g. at least 5 nm, more preferably at least 10 nm, even more preferably at least 20 nm or at least 50 nm or at least 100 nm, under the provision that $d_{cat} > 2 \times d_{SWCNT}$.

The average particle size can be determined by an image analysis (e.g. images taken from transmission electron microscopy (TEM) or scanning electron microscopy (SEM)). In this image-based particle size analysis, the size distribution of the particles shown on the TEM (or SEM) image is determined. The particle size of an individual particle is its largest extension as available from the image. The peak value of the size distribution curve is the average particle size.

The term "catalyst in the form of a film" means that the dimensions of the surface provided by the catalyst and on which the polycyclic aromatic compounds are deposited, are by far exceeding the molecular size of the aromatic compound. Preferably, such a catalyst in the form of a film provides a flat surface, thereby improving interaction between the polycyclic aromatic compound and the catalyst surface and increasing efficiency of the intramolecular cyclization.

The catalyst used in step (ii) can be crystalline, either partly or completely. However, it is also possible that the catalyst in step (ii) is amorphous.

In a preferred embodiment, a metal-containing catalyst is also used in step (i), and the metal-containing catalyst of step (i) and the metal-containing catalyst of step (ii) are the same.

The temperature $T_2$ of step (ii) can vary over a broad range, e.g. from 100 to 1000°C.

In the present invention, it was surprisingly realized that yield of SWCNTs can even be further improved when step (ii) is carried out a low temperature. So, in a preferred embodiment, step (ii) is carried out at a temperature of 700°C or less, more preferably 600°C or less, even more preferably 550°C or less. Appropriate temperature ranges are e.g. from 300 to 700°C, more preferably 350 to 600°C, even more preferably 350 to 550°C.

Preferably, the temperature of step (ii) is kept low enough so as to avoid any spontaneous formation of CNTs in step (ii). In other words, the temperature of step (ii) is preferably low enough so that the carbon-source compound is just initiating growth of the SWCNT precursor elements already provided in step (i) but does not generate further CNTs on the catalyst surface as these further CNTs would not have a well-defined uniform chirality. If the catalyst particles of step (ii) are quite large, this might already be sufficient for suppressing formation of further CNTs in step (ii). If smaller catalyst particles (but still complying with the relation $d_{cat} > 2 \times d_{SWCNT}$) are used in step (ii), there might be a remaining risk of spontaneously forming CNTs of non-uniform chirality. However, as the SWCNT precursor elements used in the present invention can react with the carbon-source compound even at low temperature, step (ii) can be carried at a temperature which is low enough for suppressing spontaneous formation of further CNTs but high enough for initiating growth of the SWCNT precursor elements provided in step (i).

The pressure at which the vapour phase reaction (and therefore SWCNT growth) are carried out in step (ii) can be varied over a broad range. The vapour phase reaction can be carried out at quite low pressure such as less than $10^{-4}$ mbar or less than $10^{-5}$ mbar or even less than $10^{-6}$ mbar but still provides a sufficient growth rate. If working at low pressure, purity of the final SWCNT might be improved. However, it is also possible to carry out the vapour phase reaction at higher pressure while still obtaining SWCNTs of high quality and extremely high isomeric purity.

In principle, step (ii) can be continued until the SWCNTs have grown to a desired length. Furthermore, if needed, the SWCNT end caps can be removed at a later stage so as to obtain open-ended SWCNTs.

If needed, the SWCNTs of the present invention can be separated from the surface of the metal-containing catalyst by methods commonly known to the skilled person.

The present invention will now be illustrated in further detail by the following Examples.

## Examples

### Characterization methods

STM measurements were performed using low temperature Scanning Tunnelling Microscope in constant current mode and sample temperature of 77 K. The system is held in a separate UHV chamber with a base pressure of $1 \times 10^{-11}$ mbar. Raman spectra were recorded in a Bruker Senterra instrument with a spectral resolution of 4 $cm^{-1}$ using a 532

and 782 nm laser with a power of 20 mW. He Ions Scanning Microscopy measurements were performed in a Carl Zeiss Orion Plus instrument with a beam energy of 30 keV and a beam current 0.4 pA.

**Preparation of polycyclic aromatic compounds**

Polycyclic aromatic compounds of Formulas (I) and (II) were prepared.

The compound of formula (I) was obtained by multistep organic synthesis according to synthetic route shown in Figure 2. 2-Bromo-6-methylbenzophenanthrene (1) was obtained by standard Wittig Olefination of 2-acetonaphtone to the respective stilbene and subsequent Mallory-photocyclization using the Katz-improvement. Suzuki coupling of **1** with 2-biphenylboronic acid gave **2.** Benzylic bromination with N-bromosuccinimide and following treatment with sodium cyanide in DMSO gave compound 4. The cyano-compound was then hydrolyzed to the corresponding acetic acid and converted to **6** in two steps. First reaction with thionylchloride gave the acid chloride which was then used in a Friedel-Crafts acylation to achieve ring closure. The last synthetic step, the conversion to compound of Formula (I) (referred to as **7** in Figure 2), was carried out by aldol cyclotrimerization using either $TiCl_4$ or Bronsted acid conditions. Both cases lead to the desired compound in satisfying yield.

The compound of formula (II) was obtained by multistep organic synthesis according to synthetic route shown in Figure 3. 2-methyl-triphenylene was prepared by Suzuki coupling of biphenyl-2-boronic acid and 4-bromotoluene resulting in **8** followed by Mallory-photocyclization using the Katz-improvement. Benzylic bromination with *N*-bromosuccinimide of **9** gave 2-bromomethyl-triphenylene which was subsequently converted to the corresponding Wittig salt **11** or phosphonate **12.** Although stilbene **13** could not be obtained by Wittig reaction of **11** and acetonaphtone, the Horner-Emmons-Wadsworth reaction using **12** and **13** was successful. The following Mallory-photocyclization resulted in formation of the desired compound **14.** The corresponding acetic acid **17** was then obtained by benzylic bromination of **14** with *N*-bromosuccinimide, a following treatment with potassium cyanide in the presence of tetrabutylammoniumbromide in $CH_2Cl_2$ and finally hydrolysis. **17** was then converted to the acid chloride by treatment with thionylchloride and subsequently Friedel-Crafts acylation was used to achieve ring closure and ketone **18** was obtained. For the trimerization of **18,** reaction with $TiCl_4$ in *o*-DCB at 180 °C was carried out. The compound of Formula (II) (referred to as **19** in Figure 3) was obtained.

**Preparation of a precursor element for (6,6) single wall carbon nanotubes by intramolecular cyclization of polycyclic aromatic compounds**

The polycyclic aromatic compound of Formula (I) and Formula (II), respectively, was evaporated in UHV from a Knudsen-cell type evaporator at a rate of 0.5 Å/min on a previously cleaned Pt(111) surface at RT.

The Pt single crystal acquired from Surface Preparation Lab (SPL) was used as a cyclodehydrogenation catalyst. Surface was cleaned by standard sputtering with Ar ions with an energy of 1 KeV, first at room temperature and then at 1100K, followed by a last flash annealing at 1370 K without ion bombardment.

A post-annealing at about 200°C was carried out. Further annealing at about 500°C induces the complete surface-catalyzed cyclodehydrogenation of the molecules, thereby forming the desired precursor element for the (6,6) SWCNT to be prepared.

The precursor element is made of a SWCNT segment and a SWCNT cap which is attached to one end of the SWCNT segment, whereas the other end of the SWCNT segment remains open. The SWCNT segment is made of a segment ring (or rather two or more rings) formed by ortho-fused benzene rings. The structure of the precursor element is shown in Figure 1. The SWCNT segment is represented by the grey shaded part, whereas the cap closing one end of the SWCNT segment is represented by the part without shading.

Figure 4 shows a STM image of the polycyclic aromatic molecules deposited on the Pt surface at RT Figure 4(a) and after Annealing at 500°C Figure 4(b). As the Pt single crystal and its surface are by far larger than the dimensions of the deposited molecules, the Pt surface reflects the situation of a more or less continuous and flat film on which the polycyclic aromatic molecules are applied.

Figure 5 shows the line profiles of a polycyclic aromatic molecule prior to cyclodehydrogenation (solid line) and the precursor element obtained after cyclodehydrogenation (dotted line). As can be seen from Figure 5, the height increases from about 0.2 nm (height of the more or less flat aromatic compound) to about 0.45 nm which is the height of the "bowl-like" precursor element.

In Figure 6, it is shown that different conformational isomers exist for the polycyclic aromatic compound of Formula (II). However, only one of these conformational isomers forms a curved bowl-like element made a SWCNT segment (in this particular case, a (6,6)-SWCNT segment) and a SWCNT cap. It is only this particular bowl-like element having the "correct" conformation which will subsequently grow in the CVD step. In Figure 6, the conformational isomer on the very left forms a precursor element (shown from two different perspectives) which consists of a (6,6)-SWCNT segment and a cap attached to one end of the tube segment.

**Growth of the precursor elements to isomerically pure (6,6) SWCNTs by vapour phase reaction with carbon-source molecules**

The carbon source compound used for growing the precursor elements to the desired (6,6) SWCNTs was ethylene ($C_2H_4$) and ethanol ($C_2H_5OH$), respectively. A pressure of $1x10^{-7}$ mbar was maintained in the chamber. The substrates were annealed at 400°C or 500°C during 1h. To have control on the low doses experiments, a pressure of $1x10^{-8}$ mbar was used.

For the CVD growth step, the precursor elements were left on the surface of the cyclodehydrogenation catalyst already used in step (i). So, the metal-containing catalyst of step (ii) was the same as used in step (i). As already mentioned above, the Pt single crystal catalyst and its surface are by far larger than the dimensions of the deposited molecules and the precursor elements prepared therefrom, which is why the Pt surface reflects the situation of a more or less continuous and flat catalyst film on which the isomerically pure SWCNTs are manufactured.

Figure 7 shows the line profiles of the SWCNT precursor element before being reacted with the carbon source compound (solid line), and after having initiated the growth phase by feeding the carbon source compound (dotted line: dose of 1 Langmuir; dashed line: dose of 5 Langmuirs). As demonstrated by Figure 7, a precursor element which consists of a (6,6)-SWCNT segment and a cap attached to one end of the tube segment will grow to the desired SWCNT by vapour phase reaction

Figure 8 shows the scanning He ions microscopy image of a SWCNT of 300 nm in length.

Figure 9 shows the SWCNT Raman spectrum and is in conformity with monodisperse isomerically pure (6,6)-SWCNTs. The Raman spectrum presents very well defined bands at the expected positions for a (6,6) SWCNT. The extremely narrow band at 295 $cm^{-1}$ is associated to the Radial Breathing Mode (RBM) of a (6,6) tube. The RBM is a tangential out-of-plane acoustic mode which frequency depends strongly on the nanotube diameter. It is extremely important to outline that the spectrum does not show any further band within the RBM range (from 200-400 $cm^{-1}$), not even under illumination with a red laser with $\lambda$=782 nm, therefore supporting the extremely high selectivity of the process. In addition, the Raman spectrum presents the same bands when illuminating much bigger areas by using a lower magnifying objective. The inset of the figure shows the RBM band recorded by illuminating an area of 80 $\mu m^2$, thus assuring the measurement of a high number of SWCNT and therefore demonstrating the high selectivity of the process. The G band associated to the in-plane optical vibration of the graphene lattice appears as a double peak at 1518 and 1591 $cm^{-1}$. The significant curvature in small diameter SWCNT causes a shift to lower frequencies in the G peaks, especially for the vibrations associated to transversal (perpendicular to the tube axis) atomic displacements ($G^-$), which appears downshifted with respect to the sp2 graphene lattice vibrations. The splitting observed is consistent with SWCNT of similar diameter and chirality. The additional peaks in the range from 400-1200 $cm^{-1}$ have been previously observed and used as an evidence for the presence of armchair SWCNT, since no peak in this range is present in semiconducting tubes.

The extremely cleanliness of the process reported here yields predefined chirality and defect-free SWCNT. A proof of that is the absence of any D band in the Raman spectrum.

A high resolution STM picture of the SWCNT structure is shown in Figure 10 which is a further proof for the true single chirality of the SWCNTs prepared in the present invention. In Figure 10, it can be observed that the SWCNTs present an internal structure consisting in higher contrast lines in the direction of the tube axis. The model of the SWCNT is superimposed over the STM picture to further corroborate that those higher contrast lines are indeed the carbon positions of the graphene structure in a (6,6) SWCNT. The outstanding agreement in both diameter of the tube and periodicity of the graphene lattice demonstrate that the 1-D structures observed are the expected (6,6) SWCNTs.

**Effect of temperature in step (ii) on product quality and yield**

Figure 11 shows the Raman spectra of (6,6)-SWCNTs prepared at a temperature of 500°C (solid line) and 400°C (dotted line) in step (ii).

Both spectra are consistent with isomerically pure (6,6)-SWCNTs. However, by comparing the relative intensities between D/G bands and RBM/G, it can be concluded that the desired pre-defined SWCNTs can be obtained at higher yield when operating at lower temperature.

**Claims**

**1.** A process for preparing single wall carbon nanotubes (SWCNT) having a diameter $d_{SWCNT}$, which comprises

(i) providing a precursor element which comprises a segment $S_{SWCNT}$ of the single wall carbon nanotube, wherein the precursor element is prepared from a polycyclic aromatic compound by a surface-catalyzed intramolecular cyclisation,
the segment $S_{SWCNT}$ being made of at least one ring formed by ortho-fused benzene rings, and having a first end E1 which is open and a second end E2 which is opposite to the first end E1,
the precursor element optionally further comprising a cap which is attached to the second end E2 of the segment $S_{SWCNT}$,
(ii) growing the precursor element by vapour phase reaction with a carbon-source compound on the surface of a metal-containing catalyst, wherein the precursor element is in contact with the surface of the metal-containing catalyst via the open end E1 of the segment $S_{SWCNT}$, and the metal-containing catalyst is in the form of particles having an average diameter $d_{cat}$ satisfying the following relation: $d_{cat} > 2 \times d_{SWCNT}$ or in the form of a continuous film, and wherein the metal-containing catalyst of step (ii) comprises a metal selected from Pd, Pt, Ru, Ir, Rh, Au, Ag, Fe, Co, Cu, Ni, or any mixture or alloy thereof.

2. The process according to claim 1, wherein the segment $S_{SWCNT}$ is made of up to 10 rings, each ring being formed by ortho-fused benzene rings.

3. The process according to one of the preceding claims, wherein the precursor element is made of the segment $S_{SWCNT}$ and the cap being attached to the second end E2 of the segment $S_{SWCNT}$.

4. The process according to one of the preceding claims, wherein the surface-catalyzed intramolecular cyclisation is a cyclodehydrogenation, cyclodehalogenation, or Bergman cyclization.

5. The process according to one of the preceding claims, wherein the surface-catalyzed intramolecular cyclisation is carried out on the surface of a metal-containing catalyst, the metal preferably being selected from Pd, Pt, Ru, Ir, Rh, Au, Ag, Fe, Co, Cu, Ni, or any mixture or alloy thereof.

6. The process according to claim 5, wherein the metal-containing catalyst of step (i) is in the form of particles having an average diameter $d_{cat}$ satisfying the following relation: $d_{cat} > 2 \times d_{SWCNT}$ or in the form of a continuous film.

7. The process according to claim 5 or 6, wherein the particles of the metal-containing catalyst in step (i) have an average particle size of at least 5 nm.

8. The process according to one of the preceding claims, wherein the precursor element is prepared at a temperature $T_1$ of from 100°C to 1000°C.

9. The process according to one of the preceding claims, wherein the carbon-source compound of step (ii) is selected from an alkane, an alkene, an alkyne, an alcohol, an aromatic compound, carbon monoxide, a nitrogen-containing organic compounds, a boron-containing organic compound, or any mixture thereof.

10. The process according to one of the preceding claims, wherein the particles of the metal-containing catalyst in step (ii) have an average particle size of at least 5 nm.

11. The process according to one of the claims 5 to 10, wherein the precursor element is grown by vapour phase reaction with the carbon-source compound on the surface of the metal-containing catalyst of step (i).

12. The process according to one of the preceding claims, wherein step (ii) is carried out at a temperature $T_2$ of 700°C or less.

**Patentansprüche**

1. Verfahren zur Herstellung von einwandigen Kohlenstoff-Nanoröhrchen (SWCNT) mit einem Durchmesser $d_{SWCNT}$, das umfasst

(i) Bereitstellen eines Vorstufen-Elements, das ein Segment $S_{SWCNT}$ von dem einwandigen Kohlenstoff-Nanoröhrchen umfasst, wobei das Vorstufen-Element aus einer polycyclischen aromatischen Verbindung durch eine Oberflächen-katalysierte intramolekulare Cyclisierung hergestellt wird,
wobei das Segment $S_{SWCNT}$ aus mindestens einem Ring, gebildet durch ortho-kondensierte Benzol-Ringe, besteht und ein erstes Ende E1, das offen ist, und ein zweites Ende E2, das zu dem ersten Ende E1 entgegengesetzt ist, aufweist,
das Vorstufen-Element gegebenenfalls weiterhin eine Kappe umfasst, die an dem zweiten Ende E2 von dem Segment $S_{SWCNT}$ befestigt ist,
(ii) Wachsenlassen des Vorstufen-Elements durch Dampf-Phasen-Reaktion mit einer Kohlenstoff-Quellen-Verbindung auf der Oberfläche von einem Metall-enthaltenden Katalysator, wobei das Vorstufen-Element mit der Oberfläche von dem Metall-enthaltenden Katalysator über das offene Ende E1 von dem Segment $S_{SWCNT}$ in Kontakt ist, und der Metall-enthaltende Katalysator in Form von Teilchen mit einem mittleren Durchmesser $d_{cat}$, der der nachstehenden Beziehung: $d_{cat} > 2 \times d_{SWCNT}$ genügt oder in Form von einem kontinuierlichen Film vorliegt, und wobei der Metall-enthaltende Katalysator von Schritt (ii) ein Metall umfasst, ausgewählt aus Pd, Pt, Ru, Ir, Rh, Au, Ag, Fe, Co, Cu, Ni oder beliebigem Gemisch oder Legierung davon.

2. Verfahren nach Anspruch 1, wobei das Segment $S_{SWCNT}$ aus bis zu 10 Ringen besteht, wobei jeder Ring durch ortho-kondensierte Benzol-Ringe gebildet wird.

3. Verfahren nach einem der vorangehenden Ansprüche, wobei das Vorstufen-Element aus dem Segment $S_{SWCNT}$ erzeugt wird und die Kappe an dem zweiten Ende E2 von dem Segment $S_{SWCNT}$ befestigt ist.

4. Verfahren nach einem der vorangehenden Ansprüche, wobei die Oberflächen-katalysierte intramolekulare Cyclisierung eine Cyclodehydrierung, Cyclodehalogenierung oder Bergman-Cyclisierung ist.

5. Verfahren nach einem der vorangehenden Ansprüche, wobei die Oberflächen-katalysierte intramolekulare Cyclisierung auf der Oberfläche von einem Metall-enthaltenden Katalysator ausgeführt wird, wobei das Metall vorzugsweise aus Pd, Pt, Ru, Ir, Rh, Au, Ag, Fe, Co, Cu, Ni oder beliebigem Gemisch oder Legierung davon ausgewählt ist.

6. Verfahren nach Anspruch 5, wobei der Metall-enthaltende Katalysator von Schritt (i) in Form von Teilchen mit einem mittleren Durchmesser $d_{cat}$, der der nachstehenden Beziehung: $d_{cat} > 2 \times d_{SWCNT}$ genügt oder in Form von einem kontinuierlichen Film vorliegt.

7. Verfahren nach Anspruch 5 oder 6, wobei die Teilchen von dem Metall-enthaltenden Katalysator in Schritt (i) eine mittlere Teilchengröße von mindestens 5 nm aufweisen.

8. Verfahren nach einem der vorangehenden Ansprüche, wobei das Vorstufen-Element bei einer Temperatur $T_1$ von 100°C bis 1000°C hergestellt wird.

9. Verfahren nach einem der vorangehenden Ansprüche, wobei die Kohlenstoff-Quellen-Verbindung von Schritt (ii) ausgewählt ist aus einem Alkan, einem Alken, einem Alkin, einem Alkohol, einer aromatischen Verbindung, Kohlenstoffmonoxid, einer Stickstoff-enthaltenden organischen Verbindung(en), einer Bor-enthaltenden organischen Verbindung oder beliebigem Gemisch davon.

10. Verfahren nach einem der vorangehenden Ansprüche, wobei die Teilchen von dem Metall-enthaltenden Katalysator in Schritt (ii) eine mittlere Teilchengröße von mindestens 5 nm aufweisen.

11. Verfahren nach einem der Ansprüche 5 bis 10, wobei das Vorstufen-Element durch Dampf-Phasen-Reaktion mit der Kohlenstoff-Quellen-Verbindung auf der Oberfläche von dem Metall-enthaltenden Katalysator von Schritt (i) gewachsen ist.

12. Verfahren nach einem der vorangehenden Ansprüche, wobei Schritt (ii) bei einer Temperatur $T_2$ von 700°C oder weniger ausgeführt wird.

**Revendications**

1. Procédé de préparation de nanotubes de carbone à paroi unique (SWCNT) ayant un diamètre $d_{SWCNT}$, qui comprend

(i) la fourniture d'un élément précurseur qui comprend un segment $S_{SWCNT}$ du nanotube de carbone à paroi unique, dans lequel l'élément précurseur est préparé à partir d'un composé aromatique polycyclique par une cyclisation intramoléculaire catalysée en surface,
le segment $S_{SWCNT}$ étant constitué d'au moins un cycle formé de cycles benzéniques ortho-condensés, et ayant

une première extrémité E1 qui est ouverte et une deuxième extrémité E2 qui est opposée à la première extrémité E1, l'élément précurseur comprenant facultativement en outre une coiffe qui est fixée à la deuxième extrémité E2 du segment $S_{SWCNT}$,
(ii) la croissance de l'élément précurseur par réaction en phase vapeur avec un composé source de carbone sur la surface d'un catalyseur contenant un métal, dans lequel l'élément précurseur est en contact avec la surface du catalyseur contenant un métal via l'extrémité ouverte E1 du segment $S_{SWCNT}$, et le catalyseur contenant un métal est sous la forme de particules ayant un diamètre moyen $d_{cat}$ satisfaisant à la relation suivante: $d_{cat} > 2 \times d_{SWCNT}$ ou sous la forme d'un film continu, et dans lequel le catalyseur contenant le métal de l'étape (ii) comprend un métal choisi parmi Pd, Pt, Ru, Ir, Rh, Au, Ag, Fe, Co, Cu, Ni, ou tout mélange ou alliage de ceux-ci.

**2.** Procédé selon la revendication 1, dans lequel le segment $S_{SWCNT}$ est constitué de jusqu'à 10 cycles, chaque cycle étant formé de cycles benzéniques ortho-condensés.

**3.** Procédé selon l'une des revendications précédentes, dans lequel l'élément précurseur est constitué du segment $S_{SWCNT}$ et la coiffe est fixée à la deuxième extrémité E2 du segment $S_{SWCNT}$.

**4.** Procédé selon l'une des revendications précédentes, dans lequel la cyclisation intramoléculaire catalysée en surface est une cyclodéshydrogénation, une cyclodéshalogénation ou une cyclisation de Bergman.

**5.** Procédé selon l'une des revendications précédentes, dans lequel la cyclisation intramoléculaire catalysée en surface est conduite sur la surface d'un catalyseur contenant un métal, le métal étant de préférence choisi parmi Pd, Pt, Ru, Ir, Rh, Au, Ag, Fe, Co, Cu, Ni, ou tout mélange ou alliage de ceux-ci.

**6.** Procédé selon la revendication 5, dans lequel le catalyseur contenant un métal de l'étape (i) est sous la forme de particules de diamètre moyen $d_{cat}$ satisfaisant à la relation suivante: $d_{cat} > 2 \times d_{SWCNT}$ ou sous la forme d'un film continu.

**7.** Procédé selon la revendication 5 ou 6, dans lequel les particules du catalyseur contenant un métal dans l'étape (i) ont une taille de particule moyenne d'au moins 5 nm.

**8.** Procédé selon l'une des revendications précédentes, dans lequel l'élément précurseur est préparé à une température $T_1$ de 100 °C à 1000 °C.

**9.** Procédé selon l'une des revendications précédentes, dans lequel le composé source de carbone de l'étape (ii) est choisi parmi un alcane, un alcène, un alcyne, un alcool, un composé aromatique, le monoxyde de carbone, des composés organiques contenant de l'azote, un composé organique contenant du bore, ou un mélange quelconque de ceux-ci.

**10.** Procédé selon l'une des revendications précédentes, dans lequel les particules du catalyseur contenant un métal dans l'étape (ii) ont une taille de particule moyenne d'au moins 5 nm.

**11.** Procédé selon l'une des revendications 5 à 10, dans lequel la croissance de l'élément précurseur est conduite par réaction en phase vapeur avec le composé source de carbone sur la surface du catalyseur contenant un métal de l'étape (i).

**12.** Procédé selon l'une des revendications précédentes, dans lequel l'étape (ii) est conduite à une température $T_2$ de 700 °C ou moins.

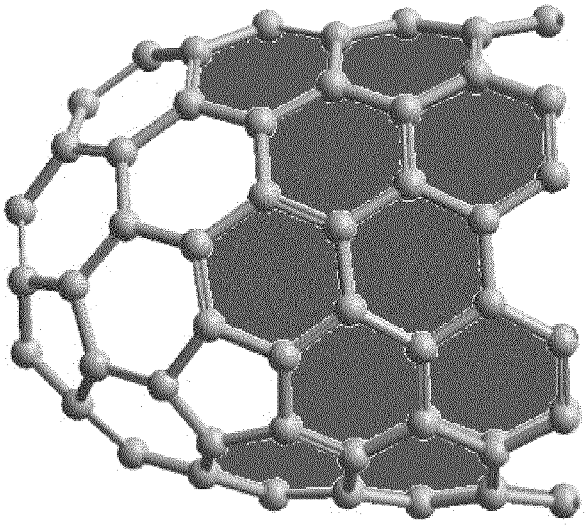

Figure 1

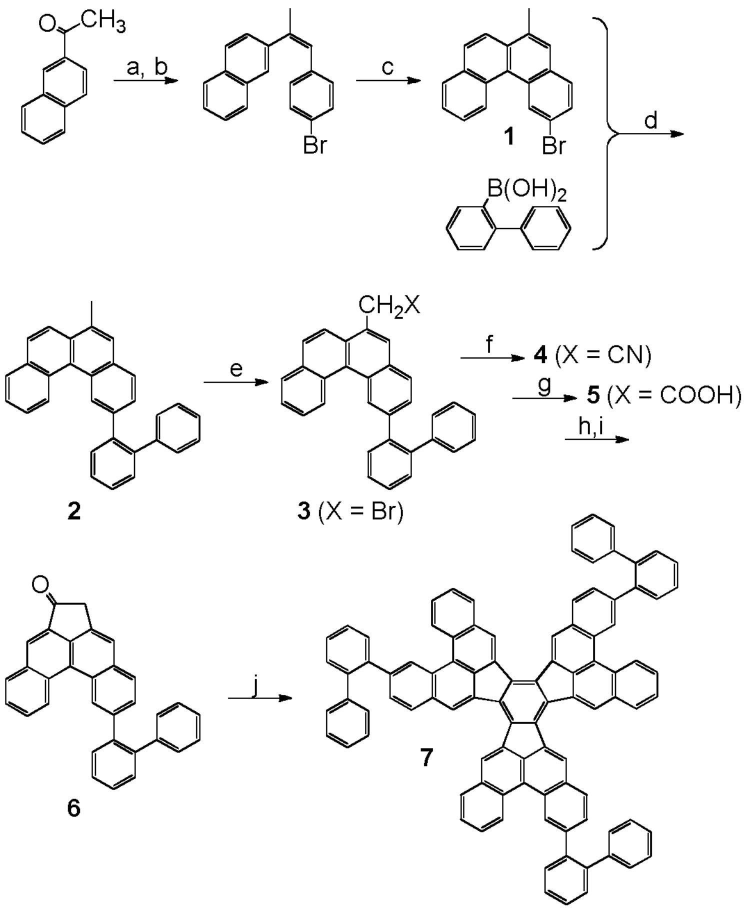


1
2
3 (X = Br)
4 (X = CN)
5 (X = COOH)
6
7

Figure 2

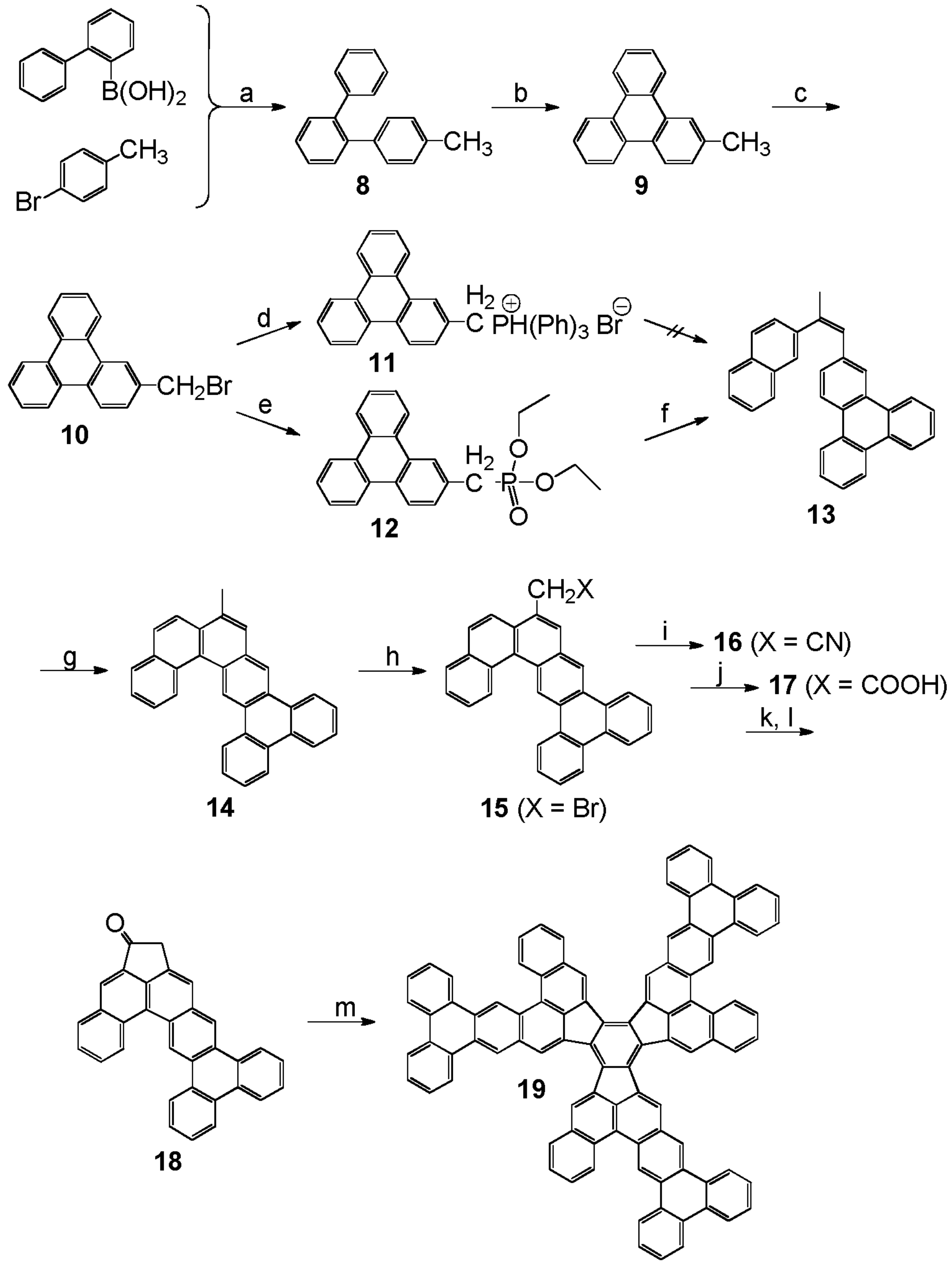


8
9
10
11
12
13
14
15 (X = Br)
16 (X = CN)
17 (X = COOH)
18
19

Figure 3

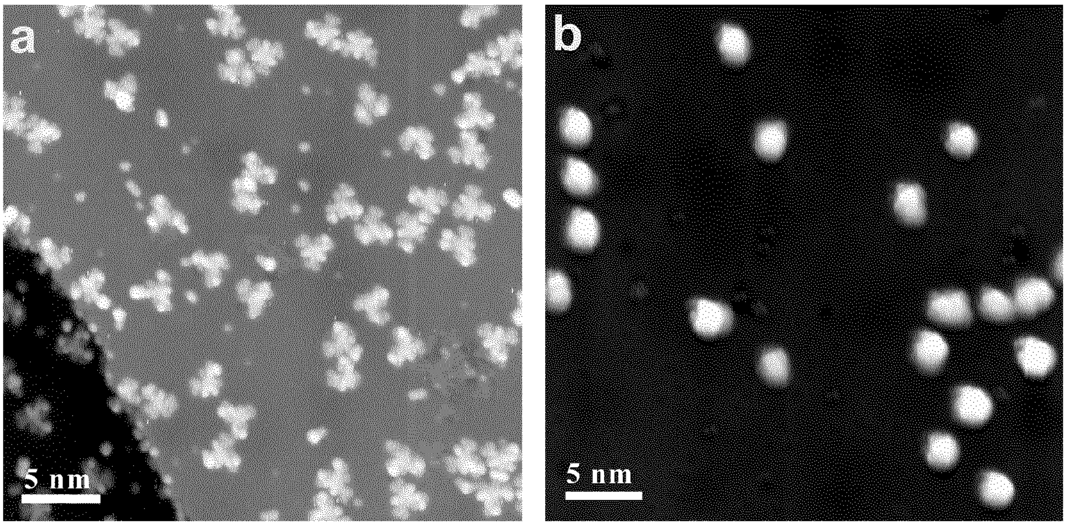


Figure 4

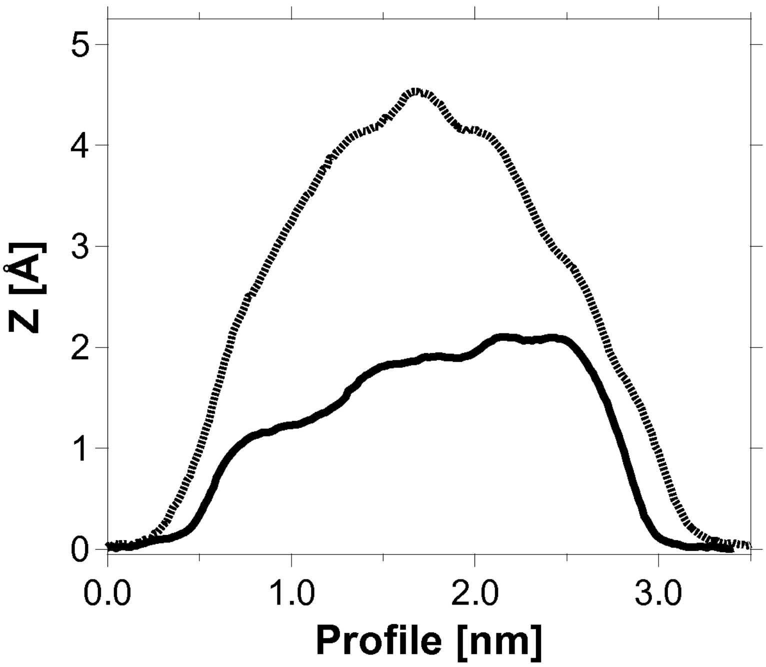


Figure 5

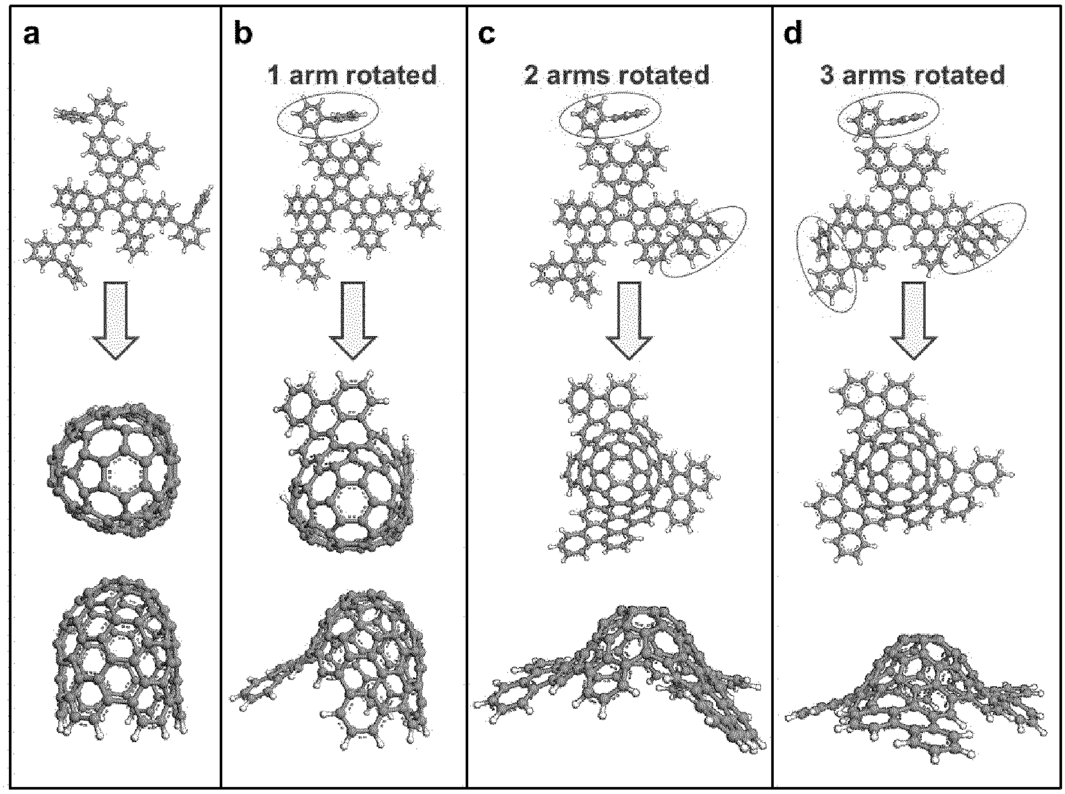


Figure 6

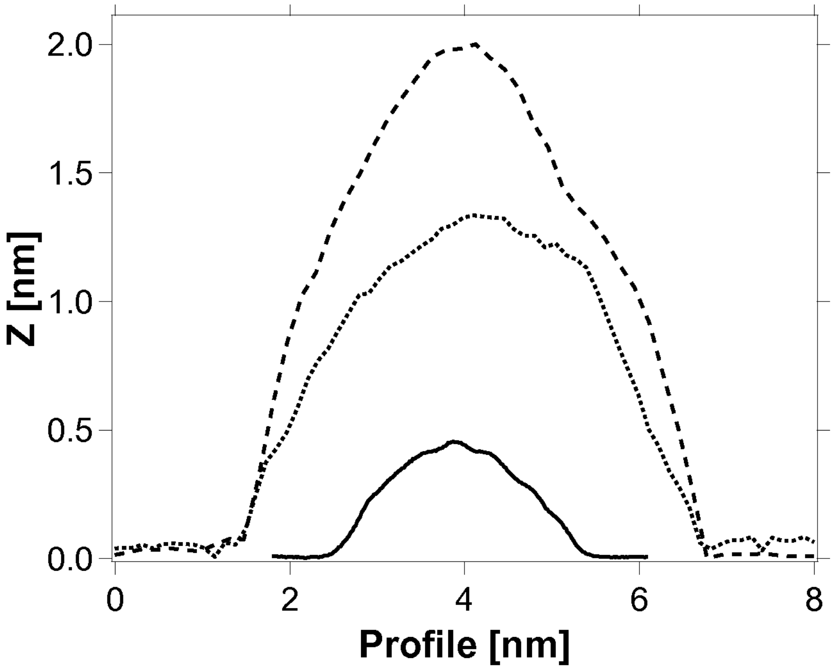


Figure 7

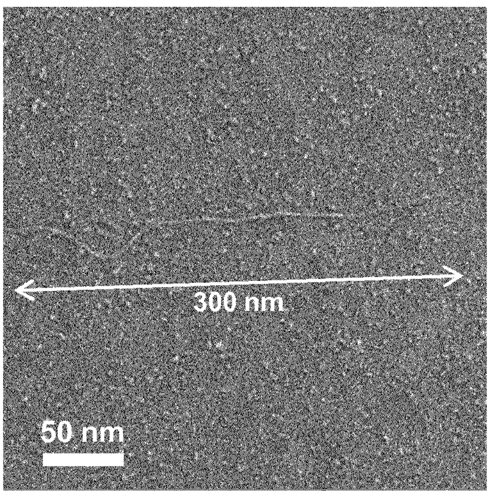


Figure 8

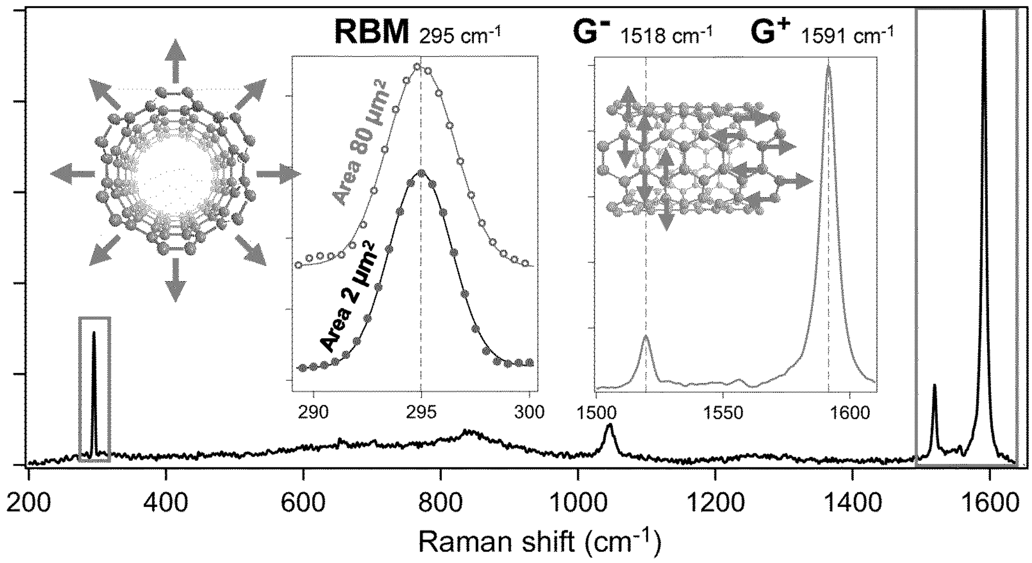


Figure 9

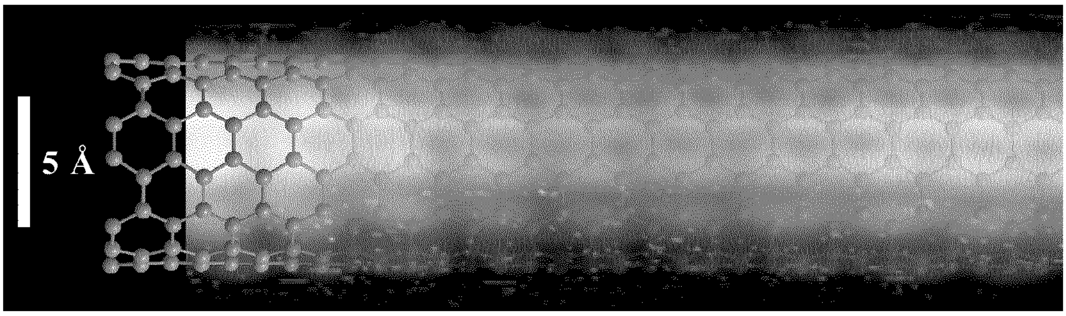


Figure 10

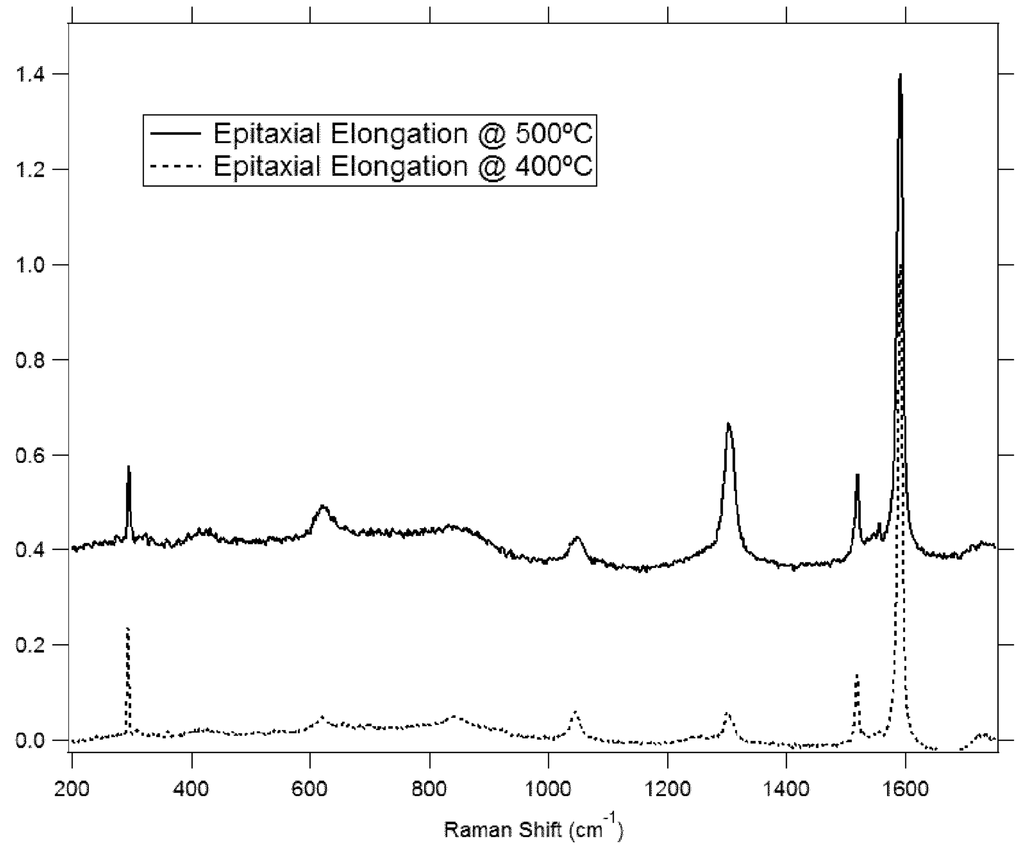

1.4
1.2
1.0
0.8
0.6
0.4
0.2
0.0
Epitaxial Elongation @ 500ºC
Epitaxial Elongation @ 400ºC
200
400
600
800
1000
1200
1400
1600
Raman Shift (cm-1)


Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120177561 A1
- EP 2684844 A1


### Non-patent literature cited in the description


- **Y. HOMMA et al.** *Nano Letters,* 2006, vol. 6 (12), 2642-2645
- **T. MARUYAMA et al.** *Mater. Express,* 2011, vol. 1 (4), 267-272
- Aromatic Belts as Sections of Nanotubes. **R. HERGES et al.** Fragments of Fullerenes and Carbon Nanotubes. John Wiley & Sons, 2012
- **K. AMSHAROV ; A. MÜLLER.** *Eur. J. Org. Chem.,* 2012, 6155-6164
- **DAISUKE TAKAGI et al.** *Nano Letters,* December 2006, vol. 6 (12), 2642-2645
- **DEBOSRUTI DUTTA et al.** *Carbon,* March 2012, vol. 50 (10), 3766-3773
- **K.Y. AMSHAROV ; A. MÜLLER.** *Eur. J. Org. Chem.,* 2012, 6155-6164
- **K. AMSHAROV et al.** *Angew. Chem. Int. Ed.,* 2010, 9392-9396
- **G. OTERO et al.** *Nature,* 2008, vol. 454, 865-868
- *Pure Appl. Chem.,* 2012, vol. 84 (4), 907-916
- *Nano Letters,* 2009, vol. 9 (4), 1673-1677